# EUROPEAN PATENT APPLICATION

(11) **EP 4 538 285 A1**
(43) Date of publication of application: **16.04.2025**
(21) Application number: 23819119.1
(22) Date of filing: 06.06.2023
(51) Int. Cl.: C07K 14/705, C07K 19/00, C12N 15/12, C12N 15/62, C12N 15/63, C12N 5/10, A61K 38/17, A61P 35/00

(54) **PD-1 VARIANT AND USE THEREOF**

(30) Priority: 06.06.2022 CN 202210632764
(71) Applicant: Chineo Medical Technology Co., Ltd., Beijing 100176 (CN)
(72) Inventor: GU, Weiyue, Beijing 100176 (CN); GAO, Bin, Beijing 100101 (CN); MEI, Xiaowei, Beijing 100176 (CN); LI, Xuelong, Beijing 100176 (CN)
(74) Representative: Winter, Brandl - Partnerschaft mbB
(86) International application number: PCT/CN2023/098627
(87) International publication number: WO 2023/236954

(57) **Abstract**

The present invention relates to a PD-1 variant and the use thereof. Specifically, the PD-1 variant of the present invention is capable of binding to PD-L1 with high affinity, but does not bind to one or more anti-PD-1 monoclonal antibodies, and preferably does not bind to at least two anti-PD-1 monoclonal antibodies. The present invention also relates to a fusion protein comprising the PD-1 variant. The present invention further relates to a coding nucleic acid molecule of the PD-1 variant and the fusion protein, and the use.

## Description

### FIELD OF THE INVENTION

The present invention relates to the field of immunotherapy. The present invention specifically provides a variant of PD-1. The present invention also provides a nucleic acid molecule that encoding the PD-1 variant, a fusion protein, or a composition comprising the PD-1 variant, as well as the uses of the PD-1 variant.

### BACKGROUND OF THE INVENTION

Programmed death protein 1, i.e. PD-1, is a protein predominantly expressed on the surface of activated lymphocytes. PD-1 is an immune checkpoint protein that has the ability to brake immune system. Accordingly, inhibition of PD-1 can unlock inhibition against the immune system to attack tumors, thus making PD-1 a popular immune target in recent years. Various antibodies against PD-1 have been approved and are currently on the market.

PD-1 binds to its ligand, PD-L1, to generate immunosuppressive signals. Tumor cells achieve immune escape through the PD-1/PD-L1 pathway by expressing PD-L1. It has been investigated that, PD-1 analogs that is capable of binding to PD-L1 have been developed to block the interaction between PD-1 and PD-L1, thereby achieving inhibition of PD-1. Alternatively, these PD-1 analogs with binding specificity to PD-L1, can also be utilized to recognize and bind tumor cells.

For example, a variety of PD-1 variant sequences are described in Stanford University's PCT patent disclosure WO2016/023001. These variants have improved affinity than human wild-type PD-1 to its ligand, PD-L1, while lack a transmembrane region. However, the variants in this application all have multiple amino acid differences compared to the wild-type. The application does not investigate the affinity of the PD-1 variants for anti-PD-1 antibodies.

When PD-1 variants are utilized for therapeutic purposes, they are sometimes combined with anti-PD-1 antibodies. In such cases, if the anti-PD-1 antibody can recognize and bind to the PD-1 variants, the two therapeutic pathways may interfere with one another, resulting in a reduced efficacy for both treatments.

Thus, there is an ongoing need in the art for enhanced PD-1 polypeptide variants for a broader range of application scenarios.

### SUMMARY OF THE INVENTION

In order to address the aforementioned problems, the inventors of the present invention constructed a library of PD-1 mutant plasmids, which were screened using flow cytometry sorting, and obtained a series of PD-1 variants. They retain a high affinity for their ligand, PD-L1, while not binding to various commercially available anti-PD-1 monoclonal antibodies. Therefore, the present invention has been achieved.

Thus, in the first aspect, the present invention provides a PD-1 variant that has a distinct amino acid sequence compared to the wild-type human PD-1 polypeptide, and, the PD-1 variant binds to PD-L1 with high affinity (for example, higher than that of wild-type human PD-1 polypeptides), while substantially not binding to a broad range of anti-PD-1 monoclonal antibodies.

In a second aspect, the present invention provides a fusion protein comprising the PD-1 variant of the first aspect. In one embodiment, the fusion protein may be a transmembrane fusion protein that includes the PD-1 variant as an extracellular segment. In one embodiment, the fusion protein is a fusion protein comprising immunoglobulin Fc.

In a third aspect, the present invention provides a nucleic acid molecule that encodes a PD-1 variant of the first aspect, or a fusion protein of the second aspect.

In a fourth aspect, the present invention provides an expression vector comprising the nucleic acid molecule of the third aspect.

In a fifth aspect, the present invention provides a host cell comprising a nucleic acid molecule as described in the third aspect or an expression vector of the fourth aspect.

In a sixth aspect, the present invention provides a pharmaceutical composition comprising a PD-1 variant of the first aspect or a fusion protein of the second aspect.

In a seventh aspect, the present invention provides for the use of the PD-1 variant of the first aspect or the fusion protein of the second aspect in the preparation of a drug. In specific embodiments, the drug is used for cancer treatment.

In an eighth aspect, the present invention provides a drug combination that includes (1) a PD-1 variant of the first aspect or a fusion protein of the second aspect, and (2) an anti-PD-1 antibody.

### BRIEF DESCRIPTION OF THE FIGURES

FIG. 1 shows a plasmid map showing a structure of the plasmid vector used to construct the PD-1 mutants library in Example 1.
FIG. 2 shows a schematic diagram showing the sorting by flow cytometry for cells binding to PD-L1 but not to anti-PD-1 antibodies in Example 3.
FIG. 3 shows the SDS-PAGE results of expressing the wild-type PD-1, or the fusion proteins of PD-1 variants with rabbit IgG Fc in Example 5. (A) non-reducing conditions; (B) reducing conditions. Lane 1 is wild-type PD-1, lanes 2-7 are PD-1 variants shown in SEQ ID Nos: 2-7, and lane 8 is a molecular weight marker.
FIG. 4 shows the blocking effect of the PD-1 variant Fc fusion protein on IL-2 release from T cells, tested in Example 7.
FIG. 5 shows that when T cells were armed with the PD-1-CD28 enhanced receptor (108-CD28) constructed with the PD-1 variant, these T cells can be stimulated by PD-L1-expressing target cells to secrete the cytokine IL-2 in Example 8.
FIG. 6 shows that the PD-1-CD28-enhanced receptor (108-CD28), constructed with the PD-1 variant, can improve the killing of corresponding target cells by T cells in Example 9.

### DETAILED DESCRIPTION OF THE INVENTION

### Definitions

Unless otherwise specified, the practice of certain methods disclosed herein employs conventional techniques from immunology, biochemistry, chemistry, molecular biology, microbiology, cell biology, genomics, and recombinant DNA technology, all of which fall within the technical scope of the art. See, for example, Sambrook and Green, "Molecular Cloning: A Laboratory Manual", 4th ed. (2012).

The term "about" or "approximately" means within an acceptable margin of error of a particular value, and the margin of error can be determined by one of ordinary skill in the art. In some cases, the acceptable margin of error depends in part on how the value is measured or determined, i.e., the limitations of the measurement system. For example, according to practice in the art, "about" may mean within 1 or more standard deviations. Alternatively, "about" may mean within a range of up to 20%, up to 10%, up to 5% or up to 1% of a given value. Alternatively, particularly with respect to a biological system or process, the term may mean within an order of magnitude of the value, preferably within 5 times the value, more preferably within 2 times the value. Where specific values are described in the present application and claims, it should be assumed that the term "about" means within an acceptable margin of error of the specific value, unless otherwise stated.

As used herein, the term "nucleotide" generally refers to a base-sugar-phosphate combination. A nucleotide may comprise a synthesized nucleotide. A nucleotide may comprise a synthesized nucleotide analog. A nucleotide may be a single unit of a nucleic acid sequence (for example, deoxyribonucleic acid (DNA) and ribonucleic acid (RNA)). The term nucleotide may include ribonucleoside triphosphates, such as adenosine triphosphate (ATP), uridine triphosphate (UTP), cytosine triphosphate (CTP), guanosine triphosphate (GTP); and deoxyribonucleoside triphosphates, such as dATP, dCTP, dITP, dUTP, dGTP, dTTP; or derivatives thereof. Such derivatives may include, for example, [αS]dATP, 7-deaza-dGTP, and 7-deaza-dATP, as well as nucleotide derivatives that confer nuclease resistance to nucleic acid molecules containing them. As used herein, the term nucleotide may refer to dideoxyribonucleoside triphosphate (ddNTP) and derivatives thereof. Illustrative examples of dideoxyribonucleoside triphosphates may include, but are not limited to, ddATP, ddCTP, ddGTP, ddITP, and ddTTP. Nucleotides may be unlabeled, or may be detectedably labeled by well known techniques. Labeling can also be performed with quantum dots. Detectable labels may include, for example, radioisotopes, fluorescent labels, chemiluminescent labels, bioluminescent labels, and enzymatic labels.

The terms "polynucleotide", "oligonucleotide" and "nucleic acid" are used interchangeably to refer to a polymerized form of nucleotides of any length, whether in the form of a single-stranded, double-stranded, or multistranded deoxyribonucleotides or ribonucleotides or their analogs. The polynucleotide may be exogenous or endogenous to the cell. The polynucleotide may be present in a cell-free environment. The polynucleotide may be a gene or a fragment thereof. The polynucleotide may be DNA. the polynucleotide may be RNA. The polynucleotide can have any three-dimensional structure and can perform any known or unknown function. The polynucleotide may comprise one or more analogs (for example, which have an altered backbone, sugar, or nucleobase). Some non-limiting examples of analogs include: 5-bromouracil, peptide nucleotides, heterogeneous nucleotides, morpholino-, locked nucleotides, glycol nucleotides, threo-nucleotides, dideoxynucleotides, cordycepin, 7-deaza-GTP, fluorophores (for example, rhodamine or fluorescein linked to a sugar), thiol-containing nucleotides, biotin- linked nucleotides, fluorescent base analogues, CpG islands, methyl-7-guanosine, methylated nucleotides, inosine, thiouracil, pseudouridine, dihydrouracil, queuosine, and wyosine. Non-restricted examples of polynucleotides include: coding or non-coding regions of genes or gene fragments, loci/locis defined by linkage analysis, exons, introns, messenger RNA (mRNA), transfer RNA (tRNA), ribosomal RNA (rRNA), short interfering RNA (siRNA), short hairpin RNA (shRNA), tiny RNA (miRNA), ribozymes, cDNA, recombinant polynucleotides, branched polynucleotides, plasmids, vectors, isolated DNAs of any sequence, isolated RNAs of any sequence, cell-free polynucleotides (including cell-free DNA (cfDNA) and cell-free RNAs (cfRNA)), nucleic acid probes and primers. The sequence of nucleotides may be interrupted by non-nucleotide components.

The term "expressing" refers to one or more processes by which a polynucleotide is transcribed from a DNA template-such as being converted into mRNA or other RNA transcripts-and/or the subsequent translation processes of the transcribed mRNA into a peptide, polypeptide, or protein.

The terms "peptide", "polypeptide" and "protein" are used interchangeably herein to refer to a polymer of at least two amino acid residues linked by one or more peptide bonds. The term does not specify a particular length for the polymer, nor does it imply or distinguish whether the peptide is produced through recombinant techniques, chemical or enzymatic synthesis, or occurs naturally. The term applies to naturally existing amino acid polymers, as well as amino acid polymers comprising at least one modified amino acid. In some embodiments, the polymer may be interrupted by a non-amino acid. The term includes amino acid chains of any length, including full-length proteins, and proteins with or without secondary and/or tertiary structures (for example, structural domains). The term also covers amino acid polymers that have been modified, for example, by disulfide bond formation, glycosylation, lipidation, acetylation, phosphorylation, oxidation, and any other operation (for example, conjugation with a labeled component). As used herein, the term "amino acid" generally refers to both natural and non-natural amino acids, including but not limited to modified amino acids and amino acid analogs. Modified amino acids may include both natural and non-natural amino acids that have been chemically modified to include a moiety or chemical portion not naturally present on the amino acid. Amino acid analogs may refer to amino acid derivatives. The term "amino acid" includes both D-amino acids and L-amino acids.

When used with respect to polypeptides, the term "variant" or "fragment" refers to a polypeptide that is related to a wild-type polypeptide, for example, by amino acid sequence, structure (for example, secondary and/or tertiary), activity (for example, enzymatic activity), and/or function. A variant and fragment of a polypeptide may comprise one or more amino acid changes (for example, mutations, insertions, and deletions), truncations, modifications, or combinations thereof, as compared to the wild-type polypeptide.

The term "fusion protein" refers to a protein created through the joining of two or more genes or fragments thereof, which were originally independent of each other. A fusion protein may comprise one or more non-natural amino acid sequences. The fusion protein may be a chimeric protein. The fusion protein may comprise a peptide affinity tag.

As used herein, the term "antigen" refers to a molecule or fragment thereof that can be bound by a selective binder. For example, an antigen can be a ligand that can be bound by a selective binder(for example, a receptor). As another example, an antigen can be an antigenic molecule that can be bound by a selective binder, such as an immune protein (for example, an antibody). Antigen may also refer to a molecule or fragment thereof that can be used in an animal to produce an antibody capable of binding to the antigen.

As used herein, the term "antibody" refers to a protein-binding molecule that has an immunoglobulin-like function. The term antibody includes antibodies (for example, monoclonal and polyclonal antibodies), as well as derivatives, variants and fragments thereof. An antibody includes, but is not limited to, immunoglobulins (Ig) of different classes (i.e., IgA, IgG, IgM, IgD, and IgE) and subclasses (for example, IgG1, IgG2, etc.). Derivatives, variants, or fragments of antibody may refer to functional derivatives, variants, or fragments that (for example, completely and/or partially) retain the binding specificity of the corresponding antibody. Antigen-binding fragments include a Fab, a Fab', a F(ab')₂, a variable fragment (Fv), a single-chain variable fragment (scFv), small antibodies, bis-antibodies, and single-domain antibodies ("sdAb" or "nano-antibodies" or "camelid antibodies (camelids)"). The term "antibody" includes antibodies and antigen-binding fragments of antibody, that have been optimized, engineered, or chemically coupled. Examples of antibodies that have been optimized include affinity matured antibodies. Examples of antibodies that have been engineered include Fc-optimized antibodies (for example, antibodies optimized in the fragment crystallizable region) and multispecific antibodies (for example, bispecific antibodies).

"Sintilimab" or "Tyvyt" is a PD-1 monoclonal antibody injection jointly developed by Innovent Biologics and Eli Lilly and Company.

"Nivolumab" or "Opdivo" is an anti-PD-1 monoclonal antibody jointly developed by Ono Pharmaceutical and Medarex company (the latter of which was purchased by Bristol Myers Squibb). Nivolumab is marketed under the trade name Opdivo, and is therefore also known as "O-drug". Opdivo is the world's first-approved and most representative anti-PD-1 monoclonal antibody.

"Tilelizumab" or "Tevimbra" is an anti-PD-1 antibody developed by BeiGene.

"Camrelizumab" or "camrelizumab" is an anti-PD-1 antibody developed by Jiangsu Hengrui.

"Pemrolizumab" or "Keytruda"is an anti-PD-1 antibody developed by Merck Sharp & Dohme. Pemrolizumab is marketed under the trade name Keytruda, and is therefore also known as "K-drug".

"Toripalimab" or "Loqtorzi" is an anti-PD-1 antibody developed by Junshi Biosciences, and is the first domestically developed anti-PD-1 antibody approved in China.

The terms "subject", "individual" and "patient" are used interchangeably herein to refer to a vertebrate, preferably a mammal, such as a human. Mammals include, but are not limited to rodents, apes and monkeys, humans, farm animals, animals in sport, and pets.

As used herein, the term "treatment" refers to a method for obtaining a beneficial or desired outcome, the beneficial or desired outcome including, but not limited to, therapeutic benefits and/or preventive benefits. Therapeutic benefits mean any treatment-related improvement or effect on one or more diseases, conditions or symptoms under treatment. For preventive benefit, the composition may be administered to a subject who is at risk of developing a particular disease, condition or symptom, or to a subject who was reported to have one or more physical symptoms of a disease, even though the disease, conditions or symptoms may not have appeared yet.

The term "effective amount" or "therapeutically effective amount" refers to an amount of an agent (for example, a variant, a fusion protein of the invention, or a composition comprising them) that is sufficient to produce the desired activity upon administration to a subject in need thereof. In the context of the text of the present disclosure, the term "therapeutically effective amount" may refer to an amount of an agent that is sufficient to delay the appearance of the condition to be treated, to halt the progression of the condition to be treated, to relieve or reduce at least one symptom of the condition to be treated.

"Synergistic effect" means that when two or more drugs are administered together, an additive or increasing effect is produced, preferably a increasing effect, i.e. the effect of the drugs, when administered together, is greater than the sum of the effects of the drugs when administered individually.

### Naming rules for mutations

In the context of the present invention, mutations present in a PD-1 variant are described with reference to the human PD-1 sequence as shown in SEQ ID NO: 1, unless otherwise specified. In the amino acid sequence of human PD-1 as shown in SEQ ID NO: 1, amino acids at positions 1-20 are the signal peptides, amino acids at positions 21-170 are the extracellular region of PD-1 , amino acids at positions 171-191 are the transmembrane region, and amino acids at positions 192-288 are the intracellular region. According to this numbering system, in the human PD-1 as shown in SEQ ID NO: 1, a extracellular region is a sequence of a total of 150 amino acids, ranging from proline at position 21 to valine at position 170, and a transmembrane region is a sequence of a total of 21 amino acids, ranging from valine at position 171 to isoleucine at position 191.

In the present invention, when describing an amino acid mutation position, it is recorded as the position of the mutated amino acid in SEQ ID NO: 1. When describing the type of amino acid mutation, the amino acid type of the corresponding amino acid position in SEQ ID NO: 1 is used as the pre-mutation amino acid and is placed before the position number (left side), and the amino acid present in the mutated PD-1 variant is placed after the position number (right side). When a single position can be mutated to different amino acids, a "/" is used to separate the multiple optional amino acids.

Based on the above rules, the A129S mutation, for example, refers to the mutation of alanine at position 129 of SEQ ID NO: 1 to serine by reference. Alternatively, A129S/H refers to the potential mutation of the alanine at position 129 to either serine or histidine.

### PD-1 variant

"PD-1 variant" in the present invention refers to a polypeptide sequence that has one or more amino acid differences compared to the wild-type human PD-1 amino acid sequence shown in SEQ ID NO: 1. In specific embodiments, a PD-1 variant of the present invention is a variant of a truncated fragment of human PD-1 wild-type, for example, a variant of a truncated fragment that does not contain an intracellular domain, or a variant that does not contain both the intracellular and transmembrane domains.

Compared to the wild-type human PD-1, the PD-1 variant of the present invention has one or more of the following characteristics:
(1) improved affinity for PD-L1 ligand,
(2) improved affinity for PD-L2 ligand, and
(3) non-binding to two or more commercially available anti-PD-1 antibodies.

In the context of the present invention, "PD-1 variant does not bind to an anti-PD-1 antibody" can mean that the binding affinity of the PD-1 variant for a particular anti-PD-1 antibody is below the limit of detection of the instrument being used or above the limit of detection of the instrument being used when affinity is expressed in terms of K_{D} values. For example, in the determination of affinity using a BIACORE T200 instrument, the K_{D} value that can be measured should be in the range of 10⁻¹² M to 10⁻³ M. When the K_{D} value is higher than 10⁻³ M, the instrument cannot detect a binding curve, i.e., the PD-1 variant as referred to herein does not bind to the anti-PD-1 antibody.

Since the most important property of the PD-1 variant of the present invention is its altered binding affinity, the mutations are primarily concentrated in the extracellular segment. Specifically, as compared to the wild-type PD-1 polypeptide, the PD-1 variant of the present invention comprises mutations at one or more amino acid positions selected from the two regions E84 to G90 and A129 to Q133. Preferably, at least one mutation is selected from the region composed of E84 to G90, and at least one mutation is selected from the region composed of A129 to Q133. Preferably, as compared to the wild-type PD-1 polypeptide, the PD-1 variant does not comprise any mutations in the region composed of M70 to K78, and has the same amino acid residues as the wild-type PD-1 polypeptide as shown in SEQ ID NO: 1 at the corresponding positions.

In a preferred embodiment, as compared to the wild-type human PD-1 polypeptide shown in SEQ ID NO: 1, the PD-1 variant of the present invention has one or more amino acid mutations selected from the group consisting of E84W, E84F; D85L, D85M; R86F; S87N, S87C, S87R, S87F, S87I; Q88L, Q88F, Q88N, Q88T; P89C, P89V, P89R; G90T, G90S; A129S, A129G, A129Q, A129Y; P130T; K131P; A132V, A132G; and Q133W.

The PD-1 variant of the present invention comprises the combinations of mutations contained in variants having the amino acid sequences shown in any of SEQ ID NOs: 2-26. Specifically, these combinations of mutations are:
(1) A129S, P130T, K131P, A132V, and Q133W;
(2) E84F, D85L, R86F, S87N, Q88L, A129S, P130T, K131P, A132V, and Q133W;
(3) E84W, D85M, R86F, S87C, Q88F, A129S, P130T, K131P, A132V, and Q133W;
(4) R86F, S87R, Q88N, P89C, G90T, A129S, P130T, K131P, A132V, and Q133W;
(5) R86F, S87F, Q88T, P89V, G90S, A129S, P130T, K131P, A132V, and Q133W;
(6) S87I, Q88N, P89R, G90S, A129S, P130T, K131P, A132V, and Q133W;
(7) E84F, D85L, R86F, S87N, Q88L, P130T, K131P, and Q133W;
(8) R86F, S87R, Q88N, P89C, G90T, A129G, P130T, K131P, and Q133W;
(9) R86F, S87R, Q88N, P89C, G90T, P130T, K131P, A132G, and Q133W;
(10) R86F, S87R, Q88N, P89C, G90T, A129Q, P130T, K131P, and Q133W; or
(11) R86F, S87F, Q88T, P89V, G90S, A129Y, P130T, K131P, and Q133W;
(12) A129H, P130F, K131R, A132F, and Q133N;
(13) A129S, P130E, K131P, A132I, and Q133V;
(14) S87L, Q88C, P89V, G90F, A129H, P130F, K131R, A132F, and Q133N;
(15) R86S, S87F, Q88E, P89V, G90R, A129H, P130F, K131R, A132F, and Q133N;
(16) R86L, S87Y, Q88L, P89K, G90Y, A129S, P130E, K131P, A132I, and Q133V;
(17) R86F, S87R, Q88N, P89C, G90T, A129W, P130T, K131P, A132M, and Q133W;
(18) R86F, S87F, Q88T, P89V, G90S, A129H, P130T, K131P, A132F, and Q133W;
(19) R86F, S87F, Q88T, P89V, G90S, A129G, P130T, K131P, A132F, and Q133W;
(20) R86F, S87F, Q88T, P89V, G90S, A129I, P130T, K131P, A132G, and Q133W;
(21) S87I, Q88N, P89R, G90S, A129T, P130T, K131P, A132F, and Q133W;
(22) S87I, Q88N, P89R, G90S, A129V, P130T, K131P, and Q133W;
(23) S87I, Q88N, P89R, G90S, P130T, K131P, and Q133W;
(24) R86F, S87R, Q88N, P89C, G90T, P130T, K131P, and Q133W;
(25) R86F, S87F, Q88T, P89V, G90S, P130T, K131P, and Q133W.

In a preferred embodiment, the PD-1 variant of the present invention comprises an amino acid sequence as shown in any of SEQ ID NOs: 2-26, or is composed of the amino acid sequence as shown in any of SEQ ID NOs: 2-26. In a preferred embodiment, the PD-1 variant of the present invention is a variant of the extracellular domain of wild-type human PD-1 as shown in SEQ ID NO: 1, which comprises an amino acid sequence as shown in any of SEQ ID NOs: 2-26, or is composed of the amino acid sequence as shown in any of SEQ ID NOs: 2-26; and which does not comprise an intracellular domain of wild-type PD-1, or does not comprise both the intracellular domain and the transmembrane domain of wild-type PD-1.

On the basis of the presence or absence of specific mutations and/or combinations of mutations as defined above, it should be understood that it may be permitted that the PD-1 variant has, at one or several amino acid positions, conserved amino acid substitutions, and that the PD-1 variants with these conserved amino acid substitutions still retain the activity and binding specificity as desired by the present invention. Substitution rules for conserved amino acid substitutions are known to those of skill in the art. For example, on the premise of comprising an amino acid combination according to any of items (1) to (25) as above and having the same amino acids as the wild-type in the region composed of M70 to K78, the PD-1 variant could be allowed to comprise one or several conserved amino acid substitutions (such as 1-20 conserved amino acid substitutions, preferably 1-15 conserved amino acid substitutions, more preferably 1-10 conservative amino acid substitutions, still more preferably 1-5 conservative amino acid substitutions) in other positions, as compared to the wild-type human PD-1.

In another embodiment, on the basis of the presence or absence of the specific mutations and/or combinations of mutations as defined above, the PD-1 variant of the present invention comprises an extracellular region having at least 85% sequence identity, preferably at least 90% sequence identity, more preferably at least 95% sequence identity, such as at least 96%, at least 97%, at least 98%, at least 99% sequence identity to the amino acid sequence shown in any of SEQ ID NOs: 2-26. Methods for determining sequence identity between two amino acid sequences are well known to those skilled in the art. For example, amino acid sequences can be compared by the BLAST program of NCBI, wherein the BLOSUM62 scoring matrix is used.

In the context of the present invention, "extracellular region" and "extracellular domain" are used interchangeably and have the same meaning, and are commonly used to describe the extracellular domain of a PD-1 variant derived from wild-type human PD-1. In any type of the above mutations, the PD-1 variant of the present invention may simply be a polypeptide fragment that corresponding to all or part of the extracellular region of wild-type PD-1, i.e., an "extracellular region variant". In other words, in this case, the PD-1 variant of the present invention can be regarded as a variant polypeptide in which the transmembrane region and the intracellular region of wild-type PD-1 have been removed, and which further comprises mutations as described above. For example, the PD-1 variant may not contain any amino acids corresponding to the intracellular region and transmembrane region of wild-type PD-1. For example, the PD-1 variant may comprise a signaling peptide located extracellularly in wild-type PD-1, such as the signaling peptide shown as amino acids 1 to 20 of SEQ ID NO: 1. For example, the exemplary PD-1 variants of the present invention are all peptides of 155 amino acids in length, which correspond to amino acids 1-155 of the wild-type human PD-1 shown in SEQ ID NO: 1. However, it is to be understood that this does not mean that the PD-1 variant of the present invention can only, or must have a length of 155 amino acids, nor does it mean that it cannot contain a transmembrane region, an intracellular region. For example, where it is desired to utilize the binding properties of the PD-1 variant of the present invention and use it to prepare fusion proteins, particularly transmembrane proteins, it is still possible to include additional amino acids corresponding to the PD-1 polypeptide, for example, by including amino acids from the transmembrane region of the wild-type PD-1 as the transmembrane region of the fusion protein.

### Fusion protein comprising PD-1 variant

Due to the unique binding properties, the PD-1 variant of the present invention, in particular the PD-1 extracellular region variant, has a broad range of application prospects. For example, the binding properties of the PD-1 variant can be utilized to target PD-L1 and/or PD-L2 (for example, to target the cells expressing PD-L1 and/or PD-L2), by using the PD-1 variant as part of a fusion protein (for example, as an extracellular binding domain).

In one embodiment, the PD-1 variant could be constructed into an antibody-like molecule. For example, the PD-1 variant could be constructed into a fusion protein directly with the Fc region of an immunoglobulin to form a functional polypeptide that specifically binds PD-L1, similar to an anti-PD-L1 antibody. Said Fc region is preferably the Fc region of an immunoglobulin of a mammal such as a human, for example the Fc region of a human IgG1.

In another embodiment, the fusion protein comprising the PD-1 variant is a chimeric stimulatory molecule, which may also be called an enhanced receptor, a switch molecule or a switch receptor. The chimeric stimulatory molecule comprises an extracellular domain, a transmembrane domain and an intracellular domain, wherein the extracellular domain is the PD-1 variant of the invention, in particular the PD-1 extracellular region variant, which could bind PD-L1 and/or PD-L2, and wherein the intracellular domain is the intracellular domain of a co-stimulatory molecule that mediating the activation of an immune cell such as a T cell. Said co-stimulatory molecules may be selected from: interleukin 2 receptor (IL-2R), interleukin 12 receptor (IL-12R), CD2, CD3, CD4, CD7, CD8, CD27, CD28, CD30, CD40, 4-1BB/CD137, ICOS, Lymphocyte function-associated antigen 1 (LFA-1), LIGHT, NKG2C or OX40. In a preferred embodiment, the co-stimulatory molecule is selected from OX40, 4-1BB, CD28, ICOS, CD27. In a particularly preferred embodiment, the co-stimulatory molecule is CD28.

The transmembrane domain of the fusion protein may come from wild-type PD-1 or a variant thereof, for example, may be the transmembrane domain itself of wild-type PD-1, or a variant thereof, or a fragment of the transmembrane domain. In one embodiment, the transmembrane domain comes from wild-type PD-1, for example comprising amino acids 171-191 of the amino acid sequence shown in SEQ ID NO: 1 or a fragment thereof, or composed of amino acids 171-191 of the amino acid sequence shown in SEQ ID NO: 1 or a fragment thereof. In one embodiment, the transmembrane domain comprises a sequence having at least 70% homology, at least 80% homology, or 90% homology to amino acids 171-191 of the amino acid sequence as shown in SEQ ID NO: 1.

The transmembrane domain of the fusion protein may also come from other sources, for example, from a transmembrane domain of co-stimulatory molecules. Immune cells can be modified to express the chimeric stimulatory molecule. Immune cells comprising the chimeric stimulatory molecule will generate an immune cell activation signal in the immune cells upon binding of PD-L1, rather than an immune cell inactivation signal typically associated with PD1/PD-L1 binding. In this way, the immunosuppressive signal is converted into an activation signal, thereby activating the function of the immune cell.

### Preparation Methods

The PD-1 variant of the present invention can be prepared by molecular biology methods.

For example, the PD-1 variant or fusion polypeptide of the invention can be prepared by recombinant techniques. Nucleotide fragments, e.g., DNA or RNA, encoding the PD-1 variant or fusion polypeptide can be synthesized in vitro, and then constructed into a suitable expression vector and expressed in a suitable host cell. For example, the sequence to be expressed can be delivered to the host cell by a viral vector, such as a lentiviral vector. Thus, the desired polypeptide is obtained by harvesting the protein expressed by the host cell and optionally purifying it.

Methods for purifying polypeptides are known in the art and can be performed, for example, using chromatographic techniques such as column chromatography, HPLC, affinity chromatography, size exclusion chromatography, and others. Additionally, the protein can be purified using magnetic beads.

For example, a PD-1 variant or fusion polypeptide can be prepared by a cell-free polypeptide synthesis system. Equipment that can be used to perform cell-free polypeptide synthesis is known in the art.

### Uses

As described above, the PD-1 variant of the present invention has a variety of potential uses due to its unique binding properties.

The PD-1 variant of the present invention retains the binding capacity to the ligands PD-L1 and PD-L2, and thus can be used to replace the function of wild-type human PD-1 or its extracellular domains in a wide variety of applications, particularly those utilizing the binding function of PD-1 to PD-L1 and PD-L2. Due to its improved binding capacity to ligands, the PD-1 variant of the present invention will even produce better results than wild-type PD-1.

In one embodiment, a chimeric stimulatory molecule comprising a PD-1 variant of the present invention, when expressed in an immune cell, can serve to convert the inhibitory PD-1/PD-L1 signaling into stimulatory signaling, thereby enhancing the function of an immune cell such as a T cell. Thus, it can be used in cell therapy using immune cells, to modify immune cells to be used as a therapeutic agent. Such therapeutic immune cells may be the subject's own immune cells, or may be derived from a donor that is semi-compatible with the subject. Said immune cells may be derived from the peripheral blood of the subject, e.g. obtained by sorting peripheral blood single nucleated cells. Said immune cells may be derived from tumor tissue, for example may be tumor infiltrating lymphocytes (TIL). In addition, T cells expressing the chimeric stimulatory molecules will increase interleukin-2 (IL-2) secretion by the T cells upon contact with PD-L1 expressing tumor cells, and interleukin-2 can in turn bind to receptors on the T cells, to further stimulate the T cells.

In one embodiment, an antibody analog comprising a PD-1 variant of the present invention has an effect similar to that of a PD-1 monoclonal antibody. For example, the fusion protein formed by the PD-1 variant of the present invention with Fc cuold block the PD-1/PD-L1 signaling pathway, thereby inhibiting the release of IL-2. Thus, the Fc fusion protein of the PD-1 mutant can play the same role as a PD-1 or PD-L1 monoclonal antibody in relieving the "immune brake".

In addition, the PD-1 variant of the present invention does not bind to a wide range of commercially available PD-1 monoclonal antibodies, this point enables its use in combination with these PD-1 monoclonal antibodies without affecting their respective potencies, thereby providing the possibility of combination. This is not possible when using wild-type human PD-1 or its extracellular region is used. Additionally, the PD-1 variant of the present invention retains the ability to bind to specific PD-1 monoclonal antibodies, making it possible to use the monoclonal antibodies for purification and other operations.

Based on the above uses, the PD-1 variant of the present invention, or a fusion protein comprising the variant, is particularly suitable for cancer treatment. "Cancer" in the context of the present invention includes malignant tumors arise in different organs, tissues and cell types, including both solid tumors and hematologic cancers.

The present invention also includes at least the items below:
1. a PD-1 variant, characterized in that:
   (1) the PD-1 variant is capable of binding to a human PD-L1 polypeptide as shown in SEQ ID NO: 28; and
   (2) the PD-1 variant does not bind to one or more anti-PD-1 antibodies selected from the group consisting of Sintilimab, Nivolumab, Camrelizumab, Pemrolizumab, and Toripalimab.
2. The PD-1 variant according to item 1, wherein
   as compared to the wild-type human PD-1 polypeptide shown in SEQ ID NO: 1, the PD-1 variant has a mutation at one or more of the following amino acid positions: E84, D85, R86, S87, Q88, P89, G90, A129, P130, K131, A132, and Q133, wherein the amino acid positions are numbered according to the amino acid sequence shown in SEQ ID NO: 1.
3. The PD-1 variant according to items 1 or 2, wherein as compared to the wild-type human PD-1 polypeptide shown in SEQ ID NO: 1, the PD-1 variant has one or more amino acid mutations selected from the group consisting of E84W, E84F; D85L, D85M; R86F; S87N, S87C, S87R, S87F, S87I, S87L, S87Y; Q88L, Q88F, Q88N, Q88T, Q88C, Q88E; P89C, P89V, P89R, P89K; G90T, G90S, G90F, G90R, G90Y; A129S, A129G, A129Q, A129Y, A129H, A129W, A129V, A129T, A129I; P130T, P130F, P130E; K131P, K131R; A132V, A132G, A132F, A132I, A132M; and Q133W, Q133N, Q133V.
4. The PD-1 variant according to item 3, wherein as compared to the wild-type human PD-1 polypeptide shown in SEQ ID NO: 1, the PD-1 variant of the present invention has one or more amino acid mutations selected from the group consisting of E84W, E84F; D85L, D85M; R86F; S87N, S87C, S87R, S87F, S87I; Q88L, Q88F, Q88N, Q88T; P89C, P89V, P89R; G90T, G90S; A129S, A129G, A129Q, A129Y; P130T; K131P; A132V, A132G; and Q133W.
5. The PD-1 variant according to any one of items 1 to 4, wherein as compared to the wild-type human PD-1 polypeptide shown in SEQ ID NO: 1, the PD-1 variant has a mutation at one or more amino acid positions selected from E84, D85, R86, S87, Q88, P89, and G90, and has a mutation at one or more amino acid positions selected from A129, P130, K131, A132, and Q133.
6. The PD-1 variant according to any one of items 1 to 5, wherein as compared to the wild-type human PD-1 polypeptide shown in SEQ ID NO: 1, the PD-1 variant comprises, or comprises only, a combination of amino acid mutations selected from any one of the following groups (1) to (25):
   (1) A129S, P130T, K131P, A132V, and Q133W;
   (2) E84F, D85L, R86F, S87N, Q88L, A129S, P130T, K131P, A132V, and Q133W;
   (3) E84W, D85M, R86F, S87C, Q88F, A129S, P130T, K131P, A132V, and Q133W;
   (4) R86F, S87R, Q88N, P89C, G90T, A129S, P130T, K131P, A132V, and Q133W;
   (5) R86F, S87F, Q88T, P89V, G90S, A129S, P130T, K131P, A132V, and Q133W;
   (6) S87I, Q88N, P89R, G90S, A129S, P130T, K131P, A132V, and Q133W;
   (7) E84F, D85L, R86F, S87N, Q88L, P130T, K131P, and Q133W;
   (8) R86F, S87R, Q88N, P89C, G90T, A129G, P130T, K131P, and Q133W;
   (9) R86F, S87R, Q88N, P89C, G90T, P130T, K131P, A132G, and Q133W;
   (10) R86F, S87R, Q88N, P89C, G90T, A129Q, P130T, K131P, and Q133W; or
   (11) R86F, S87F, Q88T, P89V, G90S, A129Y, P130T, K131P, and Q133W;
   (12) A129H, P130F, K131R, A132F, and Q133N;
   (13) A129S, P130E, K131P, A132I, and Q133V;
   (14) S87L, Q88C, P89V, G90F, A129H, P130F, K131R, A132F, and Q133N;
   (15) R86S, S87F, Q88E, P89V, G90R, A129H, P130F, K131R, A132F, and Q133N;
   (16) R86L, S87Y, Q88L, P89K, G90Y, A129S, P130E, K131P, A132I, and Q133V;
   (17) R86F, S87R, Q88N, P89C, G90T, A129W, P130T, K131P, A132M, and Q133W;
   (18) R86F, S87F, Q88T, P89V, G90S, A129H, P130T, K131P, A132F, and Q133W;
   (19) R86F, S87F, Q88T, P89V, G90S, A129G, P130T, K131P, A132F, and Q133W;
   (20) R86F, S87F, Q88T, P89V, G90S, A129I, P130T, K131P, A132G, and Q133W;
   (21) S87I, Q88N, P89R, G90S, A129T, P130T, K131P, A132F, and Q133W;
   (22) S87I, Q88N, P89R, G90S, A129V, P130T, K131P, and Q133W;
   (23) S87I, Q88N, P89R, G90S, P130T, K131P, and Q133W;
   (24) R86F, S87R, Q88N, P89C, G90T, P130T, K131P, and Q133W; and
   (25) R86F, S87F, Q88T, P89V, G90S, P130T, K131P and Q133W.
7. The PD-1 variant according to item 6, wherein
   as compared to the wild-type human PD-1 polypeptide shown in SEQ ID NO: 1, the PD-1 variant comprises, or comprises only, a combination of amino acid mutations selected from any one of the groups (1) to (11).
8. The PD-1 variant according to item 7, wherein
   as compared to the wild-type human PD-1 polypeptide shown in SEQ ID NO: 1, the PD-1 variant comprises, or comprises only, a combination of amino acid mutations selected from any one of the groups (1) to (6).
9. The PD-1 variant according to any one of items 1 to 8, wherein the PD-1 variant does not comprise an intracellular region, or does not comprise both a transmembrane region and an intracellular region.
10. The PD-1 variant according to any one of items 1 to 9, wherein the variant comprises the amino acid sequence as shown in any one of SEQ ID NOs: 2-26 or an amino acid sequence having at least 85% sequence identity to the amino acid sequence as shown in any one of SEQ ID NOs: 2-26, or is composed of the amino acid sequence as shown in any one of SEQ ID NOs: 2-26 or an amino acid sequence having at least 85% sequence identity to the amino acid sequence as shown in any one of SEQ ID NOs: 2-26.
11. The PD-1 variant according to item 10, wherein the variant comprises the amino acid sequence as shown in any one of SEQ ID NOs: 2-12 or an amino acid sequence having at least 85% sequence identity to the amino acid sequence as shown in any one of SEQ ID NOs: 2-12, or is composed of the amino acid sequence as shown in any one of SEQ ID NOs: 2-12 or an amino acid sequence having at least 85% sequence identity to the amino acid sequence as shown in any one of SEQ ID NOs: 2-12.
12. The PD-1 variant according to item 11, wherein the variant comprises the amino acid sequence as shown in any one of SEQ ID NOs: 2-7 or an amino acid sequence having at least 85% sequence identity to the amino acid sequence as shown in any one of SEQ ID NOs: 2-7, or is composed of the amino acid sequence as shown in any one of SEQ ID NOs: 2-7 or an amino acid sequence having at least 85% sequence identity to the amino acid sequence as shown in any one of SEQ ID NOs: 2-7.
13. The PD-1 variant according to any one of items 1 to 12, wherein the PD-1 variant has the same amino acids as the wild-type human PD-1 polypeptide as shown in SEQ ID NO: 1 at the following positions: M70, S71, P72, S73, N74, Q75, T76, D77 and K78.
14. The PD-1 variant according to any one of items 1 to 13, wherein the PD-1 variant has a signal peptide as shown in amino acids 1 to 20 in SEQ ID NO: 1.
15. The PD-1 variant according to any one of items 1 to 14, wherein the PD-1 variant binds to the PD-L1 polypeptide with higher affinity compared to the wild-type human PD-1 polypeptide.
16. The PD-1 variant according to any one of items 1 to 15, wherein the PD-1 variant binds to the PD-L1 polypeptide at a K_{D} value of less than 10⁻⁷ M.
17. The PD-1 variant according to any one of items 1 to 16, wherein the PD-1 variant binds to the human PD-L2 polypeptide at a K_{D} value of less than 10⁻⁶ M.
18. The PD-1 variant according to any one of items 1 to 17, wherein the PD-1 variant binds to the PD-L2 polypeptide with higher affinity compared to the wild-type human PD-1 polypeptide.
19. The PD-1 variant according to any one of items 1 to 18, wherein the PD-1 variant does not bind to at least two, preferably at least three, anti-PD-1 antibodies selected from the group consisting of Sintilimab, Nivolumab, Camrelizumab, Pemrolizumab, and Toripalimab.
20. The PD-1 variant according to item 19, wherein the PD-1 variant does not bind to the following anti-PD-1 antibodies: Sintilimab, Nivolumab and Pemrolizumab.
21. The PD-1 variant according to item 20, wherein the PD-1 variant does not bind to each of Sintilimab, Nivolumab, Camrelizumab, Pemrolizumab, and Toripalimab.
22. The PD-1 variant according to any one of items 1 to 21, wherein the PD-1 variant binds to Tilelizumab.
23. The PD-1 variant according to item 22, wherein the PD-1 variant binds to Tilelizumab with a binding affinity expressed as a K_{D} value of less than 10⁻⁹ M.
24. A fusion protein comprising the PD-1 variant according to any one of items 1 to 23.
25. The fusion protein according to item 24, wherein the fusion protein further comprises an immunoglobulin Fc fragment.
26. The fusion protein according to item 25, wherein the immunoglobulin Fc fragment is derived from a mammal, preferably a human.
27. The fusion protein according to claim 24, wherein the fusion protein comprises the PD-1 variant as an extracellular domain, wherein the PD-1 variant does not comprise an intracellular region, and the fusion protein further comprises an intracellular signal transduction domain derived from a co-stimulatory molecule.
28. The fusion protein according to item 27, wherein the co-stimulatory molecule is selected from the group consisting of OX40, 4-1BB, CD28, ICOS, CD27.
29. The fusion protein according to item 27 or 28, wherein the fusion protein further comprises a transmembrane region of PD-1.
30. An isolated nucleic acid molecule encoding the PD-1 variant according to any one of items 1 to 23.
31. The nucleic acid molecule according to item 30, wherein the nucleic acid molecule comprises the nucleotide sequence as shown in any one of SEQ ID NOs: 56-61, or the nucleotide sequence having at least 85% homology to the nucleotide sequence as shown in any one of SEQ ID NOs: 56-61.
32. An isolated nucleic acid molecule encoding the fusion polypeptide according to any one of items 24 to 29.
33. An expression vector comprising the isolated nucleic acid molecule according to any one of items 30 to 32.
34. A host cell comprising the isolated nucleic acid molecule according to items 30 or 32, or the expression vector according to claim 33.
35. A pharmaceutical composition comprising the PD-1 variant according to any one of items 1 to 23, and a pharmaceutically acceptable carrier.
36. A pharmaceutical composition comprising the fusion protein according to any one of items 24 to 29, and a pharmaceutically acceptable carrier.
37. Use of the PD-1 variant according to any one of items 1 to 23, or the fusion protein according to any one of items 24 to 29 in the preparation of a drug for the treatment of cancer.
38. A drug combination comprising (1) a PD-1 variant according to any one of items 1 to 23, or a fusion protein according to any one of items 24 to 29, and (2) an anti-PD-1 antibody.
39. The drug combination according to item 38, wherein the anti-PD-1 variant does not bind to the anti-PD-1 antibody.
40. The drug combination according to item 39, wherein the anti-PD-1 antibody is selected from the group consisting of Sintilimab, Nivolumab, Camrelizumab, Pemrolizumab, and Toripalimab.
41. The drug combination according to any one of items 38-40, which is used for the treatment of cancer.
42. A cell modified to express the fusion protein according to any one of claims 24 to 29.
43. A cell according to item 42, wherein the cell is an immune cell.
44. The cell according to item 43, wherein prior to being modified, the immune cell is derived from peripheral blood mononuclear cells or is a tumor infiltrating lymphocyte.
45. The cell according to item 44, wherein the immune cell is a T cell.
46. The cell according to any one of items 42 to 45, wherein the cell is modified for the treatment of cancer.

### Examples

For a comprehensive understanding and application of the present invention, the invention will be described in detail below with reference to embodiments and drawings, said embodiments being intended to exemplify the invention only, and not intended to limit the scope of the invention. The scope of the present invention is specifically limited by the attached items.

### Example 1. Construction of a PD-1 mutants library

This Example described the process of constructing a library of PD-1 mutants.

In summary, random mutations were performed on sites in PD-1 (SEQ ID NO: 1) that are involved in the interaction between PD-1 and PD-L1, or PD-1 and PD-L2. This was because these regions are also the regions where antibody-binding epitopes are usually located. A mutant plasmids library was thus constructed and transfected into 293T cells for subsequent analysis.

Specifically, random mutations were performed on three regions in SEQ ID NO: 1, the first region being amino acids 70-78, the second region being amino acids 84-90, and the third region being amino acids 127-133. In order to realize the random mutagenesis, 6 sets of random mutation primers were designed, and each pair of primers was capable of randomly mutating the corresponding codons of 5 consecutive amino acids within the above regions. Specifically, the 6 sets of primers contained codons for random mutagenesis of: amino acids No. 70-74 (7074-For/Rev), amino acids No. 74-78 (7478-For/Rev), amino acids No. 84-88 (8488-For/Rev), amino acids No. 86-90 (8690-For/Rev), amino acids No. 127-131 amino acids (12731-For/Rev), and amino acids 129-133 (12933-For/Rev), respectively. In the 6 sets of primers used for random mutagenesis, the upstream primers contained codons corresponding to these amino acids, and these codons were designed as 5 consecutive NNK codons, wherein N represents any of the bases A, T, C, or G, and K represents any of the bases G or T. The sequences of specific 6 sets of primers (SEQ ID NOs: 27-40) are shown in Table 1 below. Two additional primers, P1-For and P1-Rev (SEQ ID NOs: 35 and 36) were also designed, their sequences are also listed in Table 1. P1-For and P1-Rev were used in combination with the other six pairs of mutant primers to amplify the non-mutated regions and mutated regions, and were used for the amplification of DNA sequence of the complete PD-1 using overlapping PCR.

**Table 1. Primer design for random mutagenesis**

| Primer name | Primer sequences | SEQ ID NO |
|---|---|---|
| 7074-For | gtgctaaactggtaccgcNNKNNKNNKNNKNNKcagacggacaagctggccgccttc | 27 |
| 7074-Rev | gcggtaccagtttagcacgaag | 28 |
| 7478-For | taccgcatgagccccagcNNKNNKNNKNNKNNKctggccgccttccccgag | 29 |
| 7478-Rev | gctggggctcatgcggtac | 30 |
| 12731-For | tacctctgtggggccatcNNKNNKNNKNNKNNKgcgcagatcaaagagagcctg | 31 |
| 12731-Rev | gatggccccacagaggtaggtg | 32 |
| 12933-For | tgtggggccatctccctgNNKNNKNNKNNKNNKatcaaagagagcctgcgggcag | 33 |
| 12933-Rev | cagggagatggccccacagag | 34 |
| P1-For | tagctctagaccaccatgcagatcccacag | 35 |
| P1-Rev | gattgtcgacttaggagcgataggctg | 36 |
| 8488-For | caagctggccgccttccccNNKNNKNNKNNKNNKcccggccaggactgccgcttc | 37 |
| 8488-Rev | ggggaaggcggccagcttgtc | 38 |
| 8690-For | tggccgccttccccgaggacNNKNNKNNKNNKNNKcaggactgccgcttccgtgtc | 39 |
| 8690-Rev | gtcctcggggaaggcggccag | 40 |

Using the 7 sets of primer pairs in Table 1, a polymerase chain reaction was performed with PrimeStar DNA polymerase (Takara, R045B) to amplify fragments containing the PD-1 mutated region and those not containing the PD-1 mutated region, respectively. For example, to mutate amino acids 70-74, the primers used were 7074-For + P1-Rev and P1-For + 7074-Rev, respectively. Amplification conditions: 98°C for 10 s, 58°C for 5 s, and 72°C for 5 s, for a total of 30 cycles.

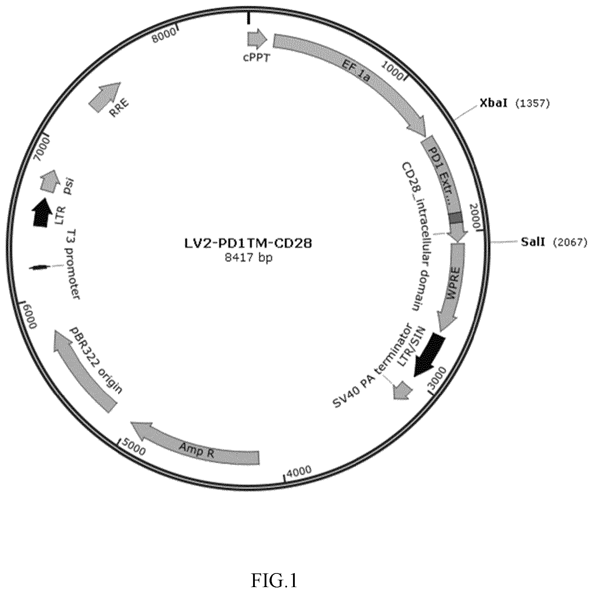

Next, overlapping PCR was performed. For instance, to mutate amino acids 70-74, a mixture of equimolar ratios of the two products amplified by the PCR described above was used as a template, the full-length sequence of PD-1 containing random mutations in amino acids 70-74 was amplified using P1-For and P1-Rev primers. Amplification conditions: 98°C for 10 s, 58°C for 5 s, and 72°C for 5 s, for a total of 30 cycles. The PCR products of the said full-length sequence and the lentiviral vector pLV2-PD1-CD28 were digested using Sal I and Xba I restriction endonucleases, respectively (the plasmid map is shown in FIG. 1; the complete sequence of the plasmid is shown in SEQ ID NO: 63, with a total of 8,417 bp, in which 1368-1877 is the PD1 extracellular region, 1878-1940 is the PD1 transmembrane region, and 1941-2063 is the CD28 intracellular region). The digested full-length sequences containing the random mutations were ligated to the vector using T4 DNA ligase, and a PD-1 random mutant plasmid library was thus prepared.

The E.coli Trans1-T1 strain (Beijing transGen biotech, Product code CD501-03) was transformed with each linkage product. Specifically, the linkage products were added to E. coli Trans1-T1 chemically transformed receptor cells thawed on ice, and placed on ice for 30 min. The cells were then incubated at 42°C for 45 seconds, after which they were placed on ice for 2 minutes and LB medium was added and incubated on a 37°C shaker for 45 minutes. After incubation, the bacterial solution was spread on LB agar plates containing ampicillin and incubated at 37°C overnight. After incubation, the total number of library clones was counted and library plasmids were extracted.

### Example 2. Transient transfection of mammalian 293T cells with PD-1 random mutant library plasmid

This Example described transfection of 293T cells with the PD-1 random mutant library plasmid constructed in Example 1, in order to construct a 293T cell line expressing a PD-1 mutant.

The 293T cells were cultured using the following method. Human embryonic kidney 293T cell line cells of less than 20 generations were taken out of liquid nitrogen, and quickly placed in a 37°C water bath pot until completely melted. The melted cells were added to 10 ml of D10 complete medium (DMEM containing 10% fetal bovine serum) medium. After well mixing, the cells were centrifuged at 400 g for 5 min and the medium was discarded. The cells were resuspended in an appropriate amount of medium, and added to T25 or T75 cell culture flasks for incubation. The starting density of cells was approximately 2 x 10° /ml. Cells were incubated at 37°C in a 5% CO₂ incubator for 72h, after which the medium was aspirated and discarded. The adherent cells were washed with phosphate buffer and the buffer was aspirated. The cells were digested with 0.5% trypsin-EDTA solution for 3-5 min on standing until most of the cells were dislodged from the bottom of the culture flask. 2mL of D10 complete medium was added and mixed. Centrifuged at 400 g for 5 min, aspirated the medium and resuspended the cells with 1 mL of D10 complete medium. The appropriate amount of cells was taken for flow cytometric reaction or passaging culture.

The constructed variant plasmid library containing random mutations in PD-1 was transfected into 293T cells by PEI (Poly(ethyleneimine), polyethyleneimine) transfection method. Each library plasmid was mixed with PEI at a mass ratio of 1:3 and transiently transfected into 293T cells. As a result, 293T cells comprising randomly mutated PD-1 variant library plasmids were obtained.

### Example 3. Flow cytometry screening for cells that bind PD-L1 but not anti-PD-1 antibodies

This Example described the process of sorting a population of cells with the desired binding capacity by flow cytometry.

After 48 hours of transient transfection, cells were digested with trypsin-EDTA solution. Centrifuged at 400 g for 5 min, aspirated the medium and resuspended the cells with phosphate buffer. A portion of the cell suspension was taken, and incubated with PD-L1-Fc (R & D Systems, 156-B7-100), Dylight650-labeled goat anti-human IgG Fc secondary antibody (Abcam, ab97006) for 30 min at 4°C. Another portion of the cell suspension was taken, then incubated with biotin-labeled anti-PD-1 antibodies (including one of Sintilimab, Nivolumab, Tilelizumab, Camrelizumab, Pemrolizumab, and Toripalimab), and PE-labeled streptavidin (BD Bioscience, 554061).

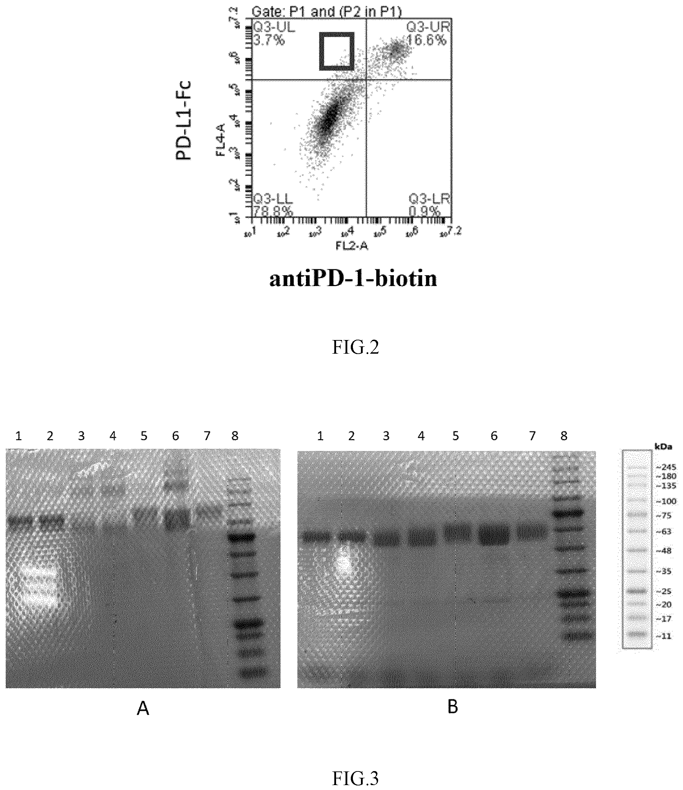

A population of cells that bind to PD-L1 and do not bind to anti-PD-1 antibodies was analyzed and collected by flow cytometric sorting. First, sorting was performed by flow cytometry using Pemrolizumab, see FIG. 2. Specifically, cells as shown in the red boxed area in the flow cytometry map of FIG. 2 were sorted. These cells were then subjected to binding assays with each of the other 5 antibodies respectively, and a population of cells that did not bind to 4 or 5 antibodies was selected. As a result of the initial screening, a total of 5,000 cells were collected.

### Example 4. Isolation and characterization of plasmids in cells obtained by sorting

This Example described a process for isolation and characterization of the PD-1 variant plasmids contained in cells obtained in Example 3.

### Sequencing

Plasmid extraction was performed on the 5000 cells sorted and collected by flow cytometry in Example 3, using the Plasmid Small Volume Extraction Kit (TransGen biotech, EM101-02).

According to the same method as in Example 1, The E. coli Trans1-T1 strain was transformed with the extracted plasmids. After incubation, a monoclonal E. coli clone was picked and sequenced with EF-1α oligonucleotide primers, to obtain the nucleotide sequence of the PD-1 variant contained in the clone, and based on this, the corresponding amino acid sequence was obtained. Of the 5,000 cells used, about 300 clones were obtained and sequenced. Of the sequenced clones, the sequencing results of a total of about 60 clones (about 20%) showed independent sequences, about 30 clones (about 10%) contained PD-1 that was successfully mutated but contained a stop codon, and about 210 clones (about 70%) contained PD-1 that was successfully mutated and could be efficiently expressed.

In Table 2, as compared to wild-type PD-1, the mutated amino acid positions and amino acid types of the screened PD-1 variants were shown (not all sequences of the sequenced clones are shown). In Table 2, SEQ ID NO: 1 was wild-type PD-1, and SEQ ID NOs: 2-26 were the sequences of representative PD-1 variants obtained.

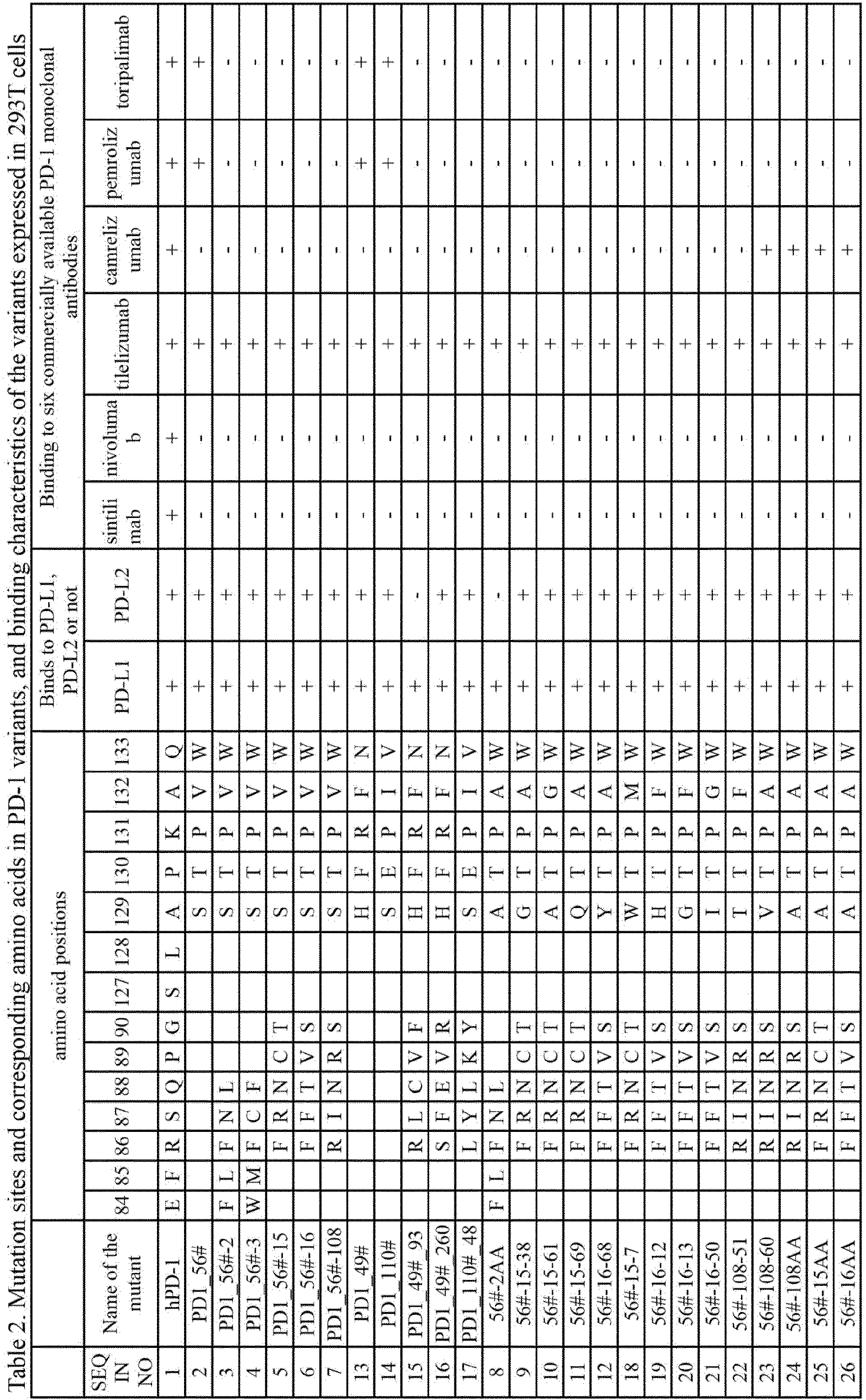

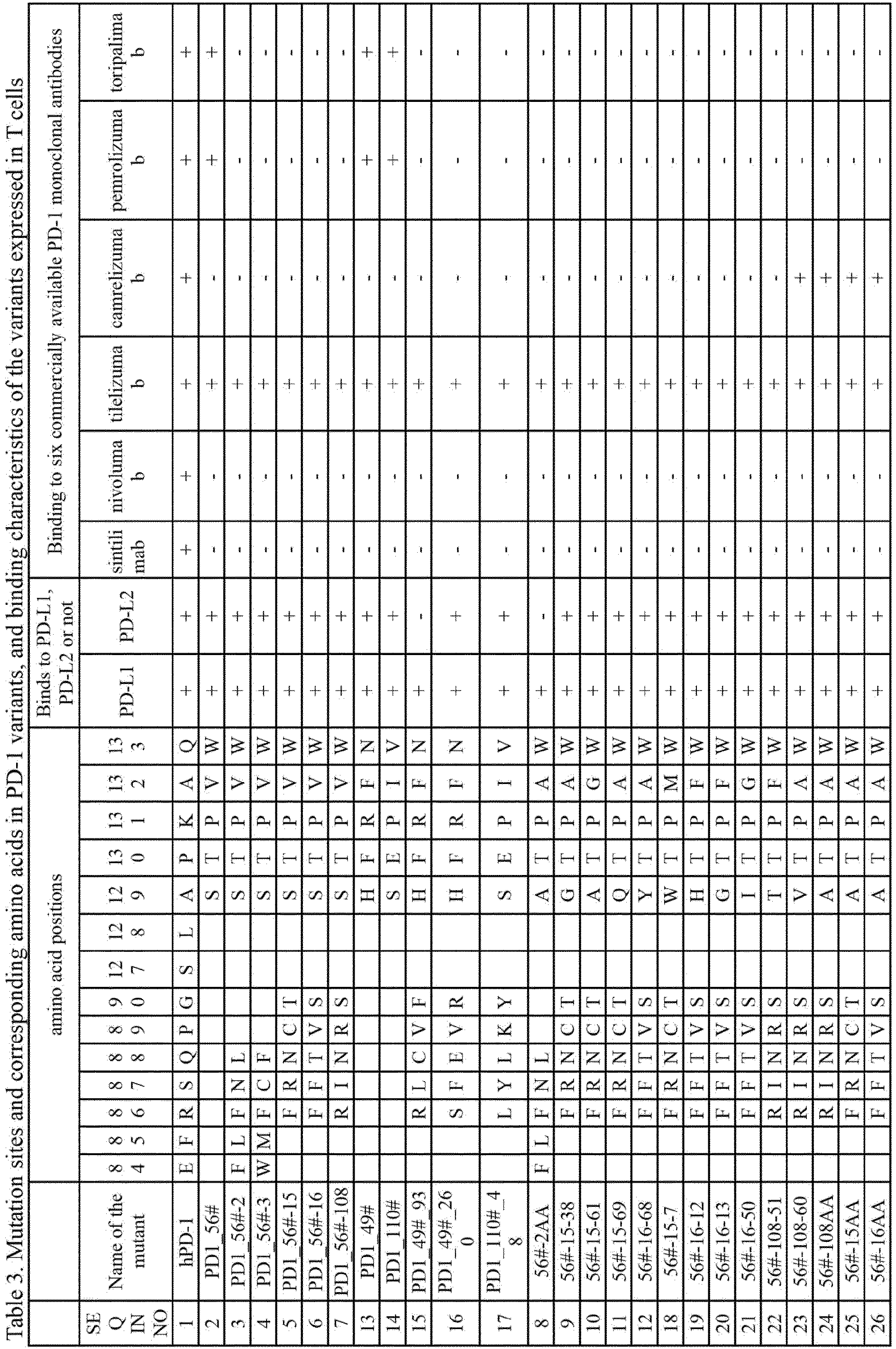

As can be seen from Table 2, the mutation sites of the variants of the present invention were concentrated in 12 sites, E84, D85, R86, S87, Q88, P89, G90, A129, P130, K131, A132, and Q133. When compared to the three regions where random mutations were performed during the initial design (see Example 1), it can be seen that these mutations are concentrated in the latter two regions, i.e., the second region from E84 to G90, and the third region from S127 to Q133 (more specifically, A129 to Q133). In each representative variant, no amino acid mutations were contained in the region from M70 to K78, and no mutation occured in S127 and L128.

### Flow cytometric analysis based on 293T cells validated the binding capacity of various mutants to PD-L1, PD-L2 and various antibodies

Based on the sequencing results, clones that as described above, were successfully mutated at the corresponding sites within the three regions expected to introduce mutations and were not mutated to the stop codon were selected. Plasmids of these clones were extracted and used to transiently transfect 293T cells, respectively. The transfection method was as described in Example 2.

Cells were digested 48 h after transfection, and a portion of the cells was taken, then incubated with a PD-L1 that fuse-expressed with an Fc derived from human IgG1 (R&D Biosystems), and a Dylight650-labeled goat anti-human IgG Fc secondary antibody (Abcam) for 30 min at 4°C. Then centrifuged at 400 g for 5 min, and the cells were resuspended and washed with PBS phosphate buffer. The centrifugation and washing were repeated once. A portion of cells was also taken and incubated with a PD-L2 that fuse-expressed with an Fc derived from human IgG1 (R&D Biosystems, PD2-H5251), and a Dylight650-labeled goat anti-human IgG Fc secondary antibody (Abcam, ab97006), and then the cells were washed twice with phosphate buffer. A portion of cells was also taken, and incubated with each of the biotin-labeled anti-PD-1 antibodies and PE-labeled streptavidin.

The binding of each of the variants as well as with wild-type PD-1 with PD-L1, PD-L2, and anti-PD-1 antibodies was analyzed by flow cytometry, and the results were shown in Table 2 (maps of raw data not shown). In Table 2, "+" represents bindable and "-" represents not bindable (i.e., below detection). The wild-type PD-1 used was a fragment of wild-type PD-1 of the same length as the variant PD-1 and at an amino acid position corresponding to positions 1 to 155 of the amino acid sequence of wild-type human PD-1 as shown in SEQ ID NO: 1.

Of the 210 clones, in which the target sequence of PD-1 was successfully mutated and did not contain a stop codon, about 60 (about 20%) were unable to bind PD-L1, about 120 (about 40%) were able to bind PD-L1 but also bound 4-6 test antibodies, and only about 30 clones met the conditions of both being able to bind PD-L1 and binding only 1-3 test antibodies (about 10%). In the end, variants featuring 25 sequences as shown in Table 2 were identified.

As shown in Table 2, based on flow cytometry analysis, wild-type human PD-1 was able to bind to PD-L1 and PD-L2 and also to all 6 tested anti-PD-1 antibodies. In contrast, all 25 representative PD-1 variants of the present invention were able to bind PD-L1; and most variants were also able to bind PD-L2 (except PD1_49#_93 and 56#-2AA). All variants were able to bind to Tilelizumab and did not bind to at least three or more anti-PD1 monoclonal antibodies. Notably, only the variants which containing mutations in the third region (PD1_56#, PD1_49#, and PD1_110#) retained the ability to bind to three antibodies (including Tilelizumab), whereas the other variants contained mutations in both the second and third regions, and retained the ability to bind to only one antibody (i.e., Tilelizumab).

### Based on flow cytometry of human T cells, the binding capacity of various mutants to PD-L1, PD-L2 and various antibodies was analyzed and verified

In the above, various mutants were expressed in 293T, and the binding capacity of various mutants to PD-L1, PD-L2 and various antibodies was tested. Given that the effector functions of various mutants were mainly realized through T cells, various mutants were further expressed in human T cells by lentivirus, and the binding capacity of the screened mutants to PD-L1, PD-L2 and various antibodies was verified by flow cytometry, and the results were shown in Table 3 (maps of raw data not shown).

The isolation, viral infection and culture of human T cells were performed as follows. 1×10⁸ peripheral blood mononuclear cells (PBMC) frozen in liquid nitrogen were taken out. After rapid thawing in a 37°C water bath, they were added to a 5-fold volume of, pre-warmed T-cell culture medium (X-VIVO15 + 0.5% HSA + 300U/ml IL-2) and mixed well. It was then centrifuged at 400 g for 5 min and the supernatant was discarded. Cells were resuspended with 20 ml of the described T-cell medium and placed in T175 culture flasks in an incubator at 37°C, 5% CO₂.

After 1 day, 3 times the cell number of CD3/CD28 magnetic beads (Thermo) were added to the cell suspension and incubation was continued.

After 1 day, MOI=3 quantity of lentivirus was added to the T cell cultures and incubation was continued. The lentivirus was prepared as follows. 0.75 mL of DMEM medium (Biological Industries, 06-1055-57-1A) and plasmid solution (VSVG 2.5 µg + dR8.91 7.5 µg + PLV2-PD1-CD28 10 µg, for a total of 20 µg) were added to tube 1. 0.75 mL of DMEM medium and 60 µl of polyethyleneimine (PEI) solution were added to tube 2. Mix the solution from tube 1 with that from tube 2 and allow to stand for 30 min, after which it was added to T75 culture flasks grown with 293T cells. The viral supernatant was collected after 24 h, then centrifuged at 100,000 g for 2 h and discarded. The virus was resuspended in 500ul X-VIVO15 medium, and used to infect T cells.

After 5 days, the complex of the cells and the magnetic beads was well blown to separate the beads from the cells as much as possible, and then the centrifuge tubes were placed on a magnetic rack for adsorption. After 1 minute, the cell suspension was transferred to a new centrifuge tube and the above blowing and adsorption steps were repeated 3 times. The cells were resuspended in T cell medium and continued to be cultured.

T cells cultured for 7-14 days were taken and incubated with PD-L1 that fuse-expressed with an Fc derived from human IgG1 (R&D Biosystems), Dylight650-labeled goat anti-human IgG Fc secondary antibody (Abcam). A portion of cells was also taken and incubated with a PD-L2 that fuse-expressed with an Fc derived from human IgG1 (R&D Biosystems, PD2-H5251-100ug) and Dylight650-labeled goat anti-human IgG Fc secondary antibody (Abcam, ab97006), and then the cells were washed twice with phosphate buffer. A portion of cells was also taken, and incubated with the biotin-labeled anti-PD-1 antibodies and PE-labeled streptavidin.

The binding of the variants with PD-1, and with anti-PD-1 antibodies was analyzed by flow cytometry, and the results were shown in Table 3. In Table 3, "+" represents bindable and "-" represents not bindable (i.e., below detection). The results in terms of binding characteristics were found to be identical to those obtained in 293T cells.

### Example 5. Preparation of PD-1 variant-rabbit IgGFc fusion protein

A part of PD-1 variants (PD-1 variants with SEQ ID NOs: 2-7) was fusion-expressed with rabbit IgG Fc and purified, and the wild-type PD-1 (SEQ ID NO: 1) was used as a control. The specific steps were as follows.

Nucleotide sequences (sequences as shown in SEQ ID NOs: 55-61) corresponding to the wild-type (SEQ ID NO: 1) and six variants (variants with amino acid sequences of SEQ ID NOs: 2-7) shown in Table 2 were amplified using PCR. The primers were P32-For and P32-Rev (SEQ ID NOs: 43 and 44) as shown below, using the pLV2-PD1-CD28 vectors constructed in Example 1 containing wild-type PD-1 and PD-1 variants as templates. Amplification reagents, systems, and amplification conditions were the same as the PCR conditions in Example 1.
P32-For: TCAGTAGCTAGCGGTACCGCCGCCACCatgcagatcccacagg
P32-Rev: GTTGAGGATCCgtgggctgtgggcacttctg

PCR products and protein expression vector pcDNA3.4-RFc (a pcDNA3.4 vector (Thermo) containing the rabbit IgGFc (RFc) gene) were digested with Nhe I and BamH I restriction endonucleases, respectively. The digested PD-1 coding sequences were ligated to the vector using T4 DNA ligase, and the pcDNA3.4-PD1-RFc expression vector was thus prepared for transfection of cells.

Before transfection, HEK293 cells (Kairuibiotech) were cultured at 37°C, 5% CO₂, 120 rpm to a density of 1×10⁶ cells/ml. The constructed pcDNA3.4-PD1-RFc vector was transfected into the HEK293 cells using the transfection reagent TA-293 (Kairuibiotech) at 5 mL/L, and the concentration of the transfection plasmid was 1.5 mg/L. The cells were cultured for 7 days after transfection at 37°C, 8% CO₂, 120 rpm. The cultures were centrifuged at 4000 rpm at the end of incubation and the supernatant was collected. Proteins were purified with Protein A beads and eluted with 500 uL of 0.1 M Gly-HCL eluent at a pH of 2.6-3.0, and the eluent was collected to obtain purified fusion proteins.

SDS-PAGE was performed on the purified fusion proteins under reducing and non-reducing conditions. The protein electropherograms are shown in FIG. 3. In FIG. 3, lanes 1-7 are the fusion proteins containing wild-type PD-1 and PD-1 variants 1-6, respectively, and lane 8 is the protein molecular weight standard. FIG. 3A shows electrophoresis under non-reducing conditions, and FIG. 3B shows electrophoresis under reducing conditions. The expected molecular weight of the fusion protein was about 43 KDa. Under reducing conditions, the size of the bands was closer to the expected. Under non-reducing conditions, the fusion protein formed a dimer and had modifications such as glycosylation, so the molecular weight was higher than 86 KDa. Under non-reducing conditions, lanes 3, 4, and 6 had distinct multiple bands, and the bands had higher molecular weights, which may indicate that the higher molecular weight bands had different forms of polymerization, such as the creation of multimers, or that there were different degrees, and different types of protein modifications.

### Example 6. Determination of affinity by Biacore T200

The affinity of wild-type PD-1 as well as the representative PD-1 variants of the present invention (variants with SEQ ID NOs: 2-7) to PD-L1, PD-L2, and the 6 PD-1 monoclonal antibodies described in Example 3 was determined by Biacore T200.

The statistical results of the affinity were shown in Tables 4A-4B. In Table 4A, "unbound" indicates that the assay result is below the lower limit of detection of Biacore T200 instrument, i.e., it indicates that the K_{D} value of the affinity is higher than 10⁻³M.

**Table 4A. Affinity assays of PD-1 mutants for PD-L1, PD-L2 and 6 PD-1 monoclonal antibodies**

| Seq No. | Affinity for PD-L1. PD-L2 K_{D} (M) | | Affinity for 6 commercially available PD-1 monoclonal antibodies K_{D}(M) | | | | | |
|---|---|---|---|---|---|---|---|---|
| | PD-L1 | PD-L2 | Sintilimab (Antibody No. 1) | Nivolumab (Antibody No. 2) | Tilelizumab (Antibody No. 3) | Camrelizumab (Antibody No. 4) | Pemrolizumab (Antibody No. 5) | Toripalimab (Antibody No. 6) |
| 1 | 8.869E-6 | 8.623E-10 | 1.414E-12 | 5.189E-10 | 1.347E-11 | 2.290E-10 | 4.689E-10 | 1.785E-10 |
| 2 | 4.565E-10 | 1.173E-9 | unbound | unbound | 8.500E-11 | unbound | 9.250E-10 | 3.946E-10 |
| 3 | 4.120E-9 | 2.962E-12 | unbound | unbound | 1.779E-9 | unbound | unbound | unbound |
| 4 | 3.956E-8 | 2.955E-7 | unbound | unbound | 2.939E-9 | unbound | unbound | unbound |
| 5 | 2.255E-9 | 4.526E-9 | unbound | unbound | 5.667E-10 | unbound | unbound | unbound |
| 6 | 6.572E-10 | 7.978E-10 | unbound | unbound | 3.146E-10 | unbound | unbound | unbound |
| 7 | 3.843E-10 | 3.364E-10 | unbound | unbound | 4.921E-11 | unbound | unbound | unbound |

**Table 4B. Raw data for Table 4A**

| Mobile phase | Stationary phase | Binding constant Ka(1/Ms) | Dissociation constant Kd(1/s) | Affinity K_{D}(M) |
|---|---|---|---|---|
| Seq No.1-RFc | hPD-L1-Fc | 1.838E+3 | 1.63E-2 | 8.869E-6 |
| Seq No.2-RFc | hPD-L1-Fc | 2.818E+6 | 1.286E-3 | 4.565E-10 |
| Seq No.3-RFc | hPD-L1-Fc | 1.011E+10 | 41.64 | 4.120E-9 |
| Seq No.4-RFc | hPD-L1-Fc | 6.735E+4 | 2.664E-3 | 3.956E-8 |
| Seq No.5-RFc | hPD-L1-Fc | 1.521E+6 | 3.431E-3 | 2.255E-9 |
| Seq No.6-RFc | hPD-L1-Fc | 2.321E+6 | 1.525E-3 | 6.572E-10 |
| Seq No.7-RFc | hPD-L1-Fc | 2.006E+6 | 7.708E-4 | 3.843E-10 |
| Seq No.1-RFc | hPD-L2-Fc | 4.067E+6 | 3.506E-3 | 8.623E-10 |
| Seq No.2-RFc | hPD-L2-Fc | 5.605E+6 | 6.574E-3 | 1.173E-9 |
| Seq No.3-RFc | hPD-L2-Fc | 4.222E+6 | 1.251E-5 | 2.962E-12 |
| Seq No.4-RFc | hPD-L2-Fc | 6.419E+3 | 1.897E-3 | 2.955E-7 |
| Seq No.5-RFc | hPD-L2-Fc | 2.120E+6 | 9.597E-3 | 4.526E-9 |
| Seq No.6-RFc | hPD-L2-Fc | 6.050E+6 | 4.827E-3 | 7.978E-10 |
| Seq No.7-RFc | hPD-L2-Fc | 1.059E+7 | 3.564E-3 | 3.364E-10 |
| Seq No.1-RFc | Anti-PD1_1# | 3.615E+6 | 5.112E-6 | 1.414E-12 |
| Seq No.1-RFc | Anti-PD1_2# | 2.342E+6 | 1.215E-3 | 5.189E-10 |
| Seq No.1-RFc | Anti-PD1_3# | 3.124E+6 | 4.207E-5 | 1.347E-11 |
| Seq No.2-RFc | Anti-PD1_3# | 2.046E+6 | 1.739E-4 | 8.500E-11 |
| Seq No.3-RFc | Anti-PD1_3# | 5.882E+5 | 1.047E-3 | 1.779E-9 |
| Seq No.4-RFc | Anti-PD1_3# | 6.912E+5 | 2.032E-3 | 2.939E-9 |
| Seq No.5-RFc | Anti-PD1_3# | 5.299E+5 | 3.003E-4 | 5.667E-10 |
| Seq No.6-RFc | Anti-PD1_3# | 2.658E+6 | 8.361E-4 | 3.146E-10 |
| Seq No.7-RFc | Anti-PD1_3# | 2.213E+6 | 1.089E-4 | 4.921E-11 |
| Seq No.1-RFc | Anti-PD1_4# | 1.700E+6 | 3.893E-4 | 2.290E-10 |
| Seq No.1-RFc | Anti-PD1_5# | 4.267E+6 | 2.001E-3 | 4.689E-10 |
| Seq No.1-RFc | Anti-PD1_6# | 6.474E+6 | 1.155E-3 | 1.785E-10 |

As can be seen from the results in Table 4, the results of the Biacore experiments validated the various binding properties determined by flow cytometry in the previous Examples, and provided specific binding affinity values. All 6 variants have high binding affinities for PD-L1 and PD-L2 as well as Tilelizumab, with K_{D} values of less than 10⁻⁶. All 6 variants had K_{D} values that were at least two orders of magnitude lower than wild-type PD-1, indicating significantly higher binding affinity for PD-L1. Variant 2 (SEQ ID NO: 3), variant 5 (SEQ ID NO: 6) and variant 6 (SEQ ID NO: 7) also have a higher binding affinity for PD-L2 than wild-type PD-1. Variant 1, variant 3 and variant 4, on the other hand, have a lower binding affinity for PD-L2 than wild-type PD-1. In terms of binding affinity for the anti-PD-1 antibody Tilelizumab, variant 1 (SEQ ID NO: 2) and variant 6 (SEQ ID NO: 7) are comparable to wild-type PD-1.

### Example 7. Functional assay of inhibition of cytokine secretion by 108-CD28Fc

When PD1-CD28-expressing T cells (PD1-CD28-T) were co-cultured with PD-L1-positive tumor cells (J82-PD-L1), the T cells released IL-2. With the addition of anti-PD-1 antibody, the anti-PD-1 antibody would bind PD-1, thereby blocking T cells from binding to tumor cells via PD-1, and thus inhibiting the release of IL-2. Similarly, when the PD-1 mutant Fc fusion protein was added, it binds to PD-L1 on the target cell so that PD-L1 cannot bind to PD-1 on the T cell membrane, and also has a similar effect of blocking IL-2 release as the anti-PD-1 antibody.

### Preparation process of PD1-CD28-T cells

The pLV2-PD1-CD28 plasmid (wherein the PD-1 is human wild-type PD-1) described in Example 1 was prepared into a lentiviral vector as described in Example 4, and used to infect human T cells to obtain PD1-CD28-T cells. The T cell culture method was described in Example 4.

### Preparation process of J82-PDL1 cells

The pLV2-PDL1 plasmid (wherein the PD-L1 is human wild-type PD-L1) was prepared into a lentiviral vector as described in Example 4, and used to infect a human bladder cancer cell line (purchased from the Chinese Academy of Medical Sciences, Product code 1101HUM-PUMC000346) to obtain the J82-PDL1 cell line.

### Assay of IL-2 secretion inhibition by PD-1 mutant Fc fusion protein

1. J82-PDL1 cells were digested with 0.05% trypsin, washed and resuspended as described in Example 2, with the density adjusted to 1 × 10⁵/mL. PD1-CD28-T cells were taken, washed, resuspended, and the density was adjusted to 1 × 10⁶/mL as described in Example 4, and then add 6 ng/mL of PMA (Phorbol 12-tetradecanonate 13-acetate, Sigma, Product code P8139-1MG).
2. 100ul of J82-PDL1 cells and 100ul of PD1-CD28-T cells were added to a 96-well plate respectively, and final concentrations of 0.8, 1.6, 3.2, 6.4, 12.8, 25.6, and 51.2 nM of Anti-IgG4-RFc (negative control, does not bind to PD-1 or PD-L1), Tilelizumab (positive control), and the 2 PD1-RFc (variants 56#-16, 56#-108) fusion proteins prepared in Example 5 were added respectively, and incubated at 37°C for 16 hours.
3. The 96-well plate was centrifuged at 500g×3min, and 50 µL of supernatant was taken to detect concentration of IL-2 by IL-2 ELISA kit (Dakewe Biotech Co., Ltd., Product code 1110203).

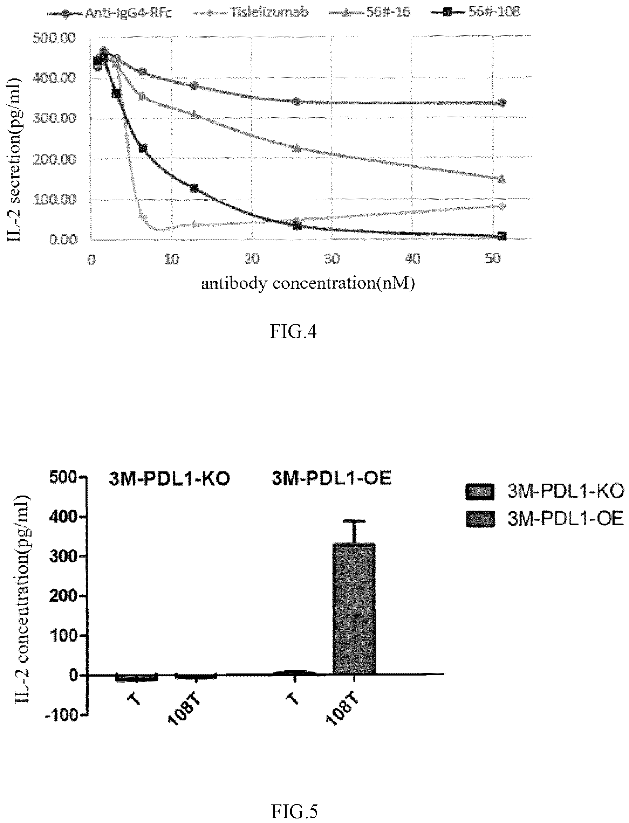

Table 5 showed the measured values of the concentration of IL-2 secreted by T cells after the addition of different concentrations of antibodies or fusion proteins, and FIG. 5 showed the trend chart of the data in Table 5. The data in Table 5 indicated that for Tilelizumab, there was no significant change in IL-2 secretion at concentrations lower than 6.4 nM, and IL-2 secretion was significantly inhibited when the concentration was ≥6.4 nM; for the 56#-16 fusion protein, IL-2 secretion tended to decrease with increasing concentration, but IL-2 secretion was not completely inhibited; and for the 56#-108 fusion protein, IL-2 secretion showed a decreasing trend with increasing concentration, and the trend was more obvious than that of 56#-16 fusion protein group and less obvious than that of Tilelizumab group, but the inhibition effect of IL-2 secretion was stronger than that of Tilelizumab group at the concentration of ≥25.6 nM. This result suggests that the PD-1 mutant fusion protein has a similar effect of blocking PD-1/PD-L1 signaling as PD-1 monoclonal antibody.

**Table 5**

| Antibodies/PD1-Fc Mol concentration (nM) | IL-2 concentration (pg/ml) | | | |
|---|---|---|---|---|
| | Rab anti hu FC (Anti-IgG4-RFc) | 3#antibody (Tislelizumab) | PD1variant 5 (56#-16) | PD1 variant 6 (56#-108) |
| 0.8 | 426.65 | 432.41 | 451.46 | 442.86 |
| 1.6 | 466.29 | 439.62 | 443.56 | 448.12 |
| 3.2 | 449.17 | 439.62 | 434.79 | 363.39 |
| 6.4 | 414.58 | 56.11 | 355.32 | 225.49 |
| 12.8 | 380.88 | 36.92 | 309.88 | 126.72 |
| 25.6 | 341.24 | 47.73 | 227.25 | 33.91 |
| 51.2 | 335.84 | 80.52 | 147.77 | 4.79 |
| 102.4 | 310.61 | 126.11 | 96.72 | -3.46 |

### Example 8. Assay of cytokine secretion

In this Example, the effect of an enhanced receptor based on the PD1 variant of the present invention on T cell function was tested by an assay of IL-2 secretion.

When PD1-CD28-expressing T cells (PD1-CD28-T) were co-cultured with PD-L1-positive tumor cells, the T cells would release the cytokine IL-2 through the interaction of PD-1 with PD-L1. Therefore, the amount of IL-2 in the co-cultured culture medium detected by enzyme-linked immunosorbent assay (ELISA) can be determined to determine the effect of PD1-CD28 in promoting IL-2 secretion.

As shown in FIG. 5, in a 96-well plate, Malme-3M tumor cells overexpressing human PD-L1 (3M-PDL1-OE) and Malme-3M tumor cells knocked out of human PD-L1 (3M-PDL1-KO) were used as target cells, and T cells expressing an enhanced receptor consisting of PD1 variant (56#-108 mutant) and CD28 (shown as 108T in FIG. 5, which can also be referred to as 108-CD28-T) and T cells without genetic modification (T) were used as effector cells, respectively. The cells were co-cultured in medium supplemented with 3 ng/ml PMA (phorbol ester) at a ratio of target cells: 108T = 10 : 1, i.e., 1 × 10⁵/well for target cells and 1 × 10⁴/well for 108T cells. The supernatants from overnight culture were used as samples to be tested, and analyzed by IL-2 ELISA along with assay standards.

ELISA was performed using a human IL-2 detection kit (ELISA method) (Dakewe Biotech Co., Ltd., Product code 1110203) according to the following procedure: The microtiter plate was pre-coated with anti-human IL-2 capture antibody. To the microtiter plate, standards or samples to be tested and detection antibody (biotin-labeled anti-human IL-2 antibody) were added at the same time, and then horseradish peroxidase-labeled streptavidin (SA-HRP), which specifically binds biotin in the immune complex, was added. The enzyme substrate tetramethylbenzidine (TMB) was added, and blue color appeared, the shade of the color correlated with the concentration of IL-2 in the standards or samples. After 5-10 minutes of color development, the reaction was terminated by adding a termination solution, and the light absorption value (OD) at 450 nm was read by a multi-mode microplate reader. The concentration of human IL-2 was proportional to the OD value within a certain range, and the standard curve was obtained by ELISA using IL-2 standard. The IL-2 concentration in each co-culture was calculated using this standard curve.

As shown in FIG. 5, in the case of target cells with knockdown of PD-L1, due to the absence of a stimulatory source of the second signal, neither control T cells nor T cells armed with PD-1 variant enhanced receptor (108T, i.e., 108-CD28-T) secreted IL-2(red bar graph); in contrast, when co-cultured with target cells highly expressing PD-L1, T cells armed with the 108T enhanced receptor released large amounts of IL-2 upon stimulation, suggesting that the fusion protein of PD1 variant 108 with CD28 (108-CD28) has a function in transmitting a signal to stimulate T cells.

### Example 9. Tumor cell killing assay

This Example related to cell killing assays. By enabling T cells to express a PD1-C28 enhanced receptor comprising a PD-1 variant of the present invention, it was demonstrated that this PD-1 variant-enhancing receptor could enhance the kill ability of T cells for tumor cells having PD-L1 expression.

Tumor cells can express HLA-peptide complex antigens on their surface, and T cells can express T cell receptors (TCRs) that target this antigen. By co-incubating such a tumor cell with its corresponding T cell, the contact between the TCR on the T cell and the complex antigen on the tumor cell will mediate a series of T cell killing responses to lyse the tumor cell.

In this experiment, the tumor cells were J82 cells expressing Eso1 antigen and PD-L1 (J82Eso1PDL1). To prepare the J82Eso1PDL1 tumor cell line, lentiviral vectors expressing the human PD-L1 and ESO1 genes were prepared according to the method in Example 4, named pLV2-PDL1 and pLV2-ESO1, respectively. A human bladder cancer cell line (purchased from the Institute of Basic Medical Sciences, Chinese Academy of Medical Sciences, Product code 1101HUM-PUMC000346) was infected with said pLV2-PDL1 lentivirus, as described in Example 4, thereby obtaining J82-PDL1 cells expressing human PD-L1. Then, the J82-PDL1 cells were then infected with pLV2-ESO1 lentivirus prepared as described above. The ESO1 antigen of this cell, after being expressed and processed intracellularly, was delivered extracellularly as ESO1peptide, and formed an antigen complex with the HLA*A0201 protein expressed on the surface of the J82 cell, the antigen complex can be recognized by a specific T cell receptor (T cell receptor, TCR).

Three different T cells were prepared, including: (1) T cells expressing a TCR targeting the HLA-Eso1 peptide (TCR-T); (2) T cells expressing an enhanced receptor consisting of a PD1 variant (56#-108 mutant, i.e., variant 6) and CD28, as well as a TCR targeting the HLA-Eso1 peptide (108-TCRT); and (3) as a control, T cells expressing the GFP green fluorescent protein T cells (GFP-T).

First, PD1+ T cells were prepared. Frozen whole blood cells from healthy donors were taken out and resuscitated rapidly in a water bath at 37°C and added to 5 times the volume of pre-warmed T cell culture medium. The medium was X-VIVO15 (Lonza; BE02-053Q) containing 0.5% human serum albumin (Xin Ji Er, Beijing SL Pharmaceutical Co. Ltd.). After well mixing, the cells were centrifuged at 400g for 5 min, and the supernatant was removed. The cells were resuspended with PBS-0.5% HSA to make a density of 10⁸ total cells/ml. Biotin-labeled anti-PD1 antibody Biotin-anti-PD1 (Biolegend, Product code 329934) was added, and incubated at 4°C for 20 min, and washed twice with PBS-0.5% HSA. Anti-Biotin Microbeads (Anti-Biotin Microbeads) (Miltenyi Biotec, Product code 130-090-485) were added, mixed well, and incubated at 4°C for 20 min. Washed twice with PBS-0.5% HSA. PD1+ T cells were sorted using an LS sorting column (Miltenyi Biotec, Product code 130-042-401). The obtained PD1+ T cells were cultured with reference to the manner described in Example 4.

To prepare TCR-T cells, a lentiviral vector pLV2-TCR comprising the coding sequence of a TCR was prepared as described in Example 4. The TCR is a T-cell receptor specifically recognizing the HLA*A0201/ES01peptide complex, and the coding sequence is shown in SEQ ID NO: 41. Next, according to the method described in Example 4, PD1 + T cells were infected using the lentiviruse, and TCR-T cells were obtained.

In order to prepare T cells expressing the enhanced receptor, lentiviral vectors containing the coding sequence of PD1 mutant enhanced receptor PD1-CD28 (abbreviated as 108-CD28) and TCR were produced according to the method described in Example 4. The lentivirus was then used to infect the above-described PD1+ T cells, which were infected as described in Example 4, and 108-TCR T cells were obtained. The gene sequence of 108-TCR is shown in SEQ ID NO: 42. The transmembrane region in the enhanced receptor is a transmembrane region derived from PD1.

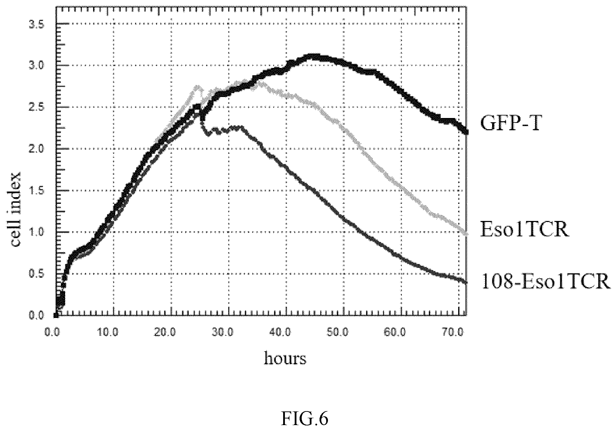

Killing assays were performed using real-time, label-free, dynamic cell analysis (RTCA) to detect the survival of target cells. Specifically, J82Eso1PDL1 target cells were first cultured in 96-well plates in culture plates (100ul per well, target cell concentration 8x10⁴/ml). At the time point of 18 hours, TCR-T cells or TCR-T cells modified with enhanced receptor (108-TCR-T) were added to the corresponding wells, respectively, and the growth of the target cells was observed and recorded, and the results were shown in FIG. 6.

In FIG. 6, the cell index in the vertical coordinate reflected the adherence situation of the cells, with higher values indicating more adherent cells, which also means more live cells and good growth. The horizontal coordinate was time, expressed in hours.

As seen in FIG. 6, TCR-T cells which expressing the enhanced receptor all showed stronger inhibition of tumor cells than TCR-T alone. J82Eso1PDL1 co-cultured with GFP-T, a control cell without TCR, maintained its growth state despite some non-specific killing effects (FIG. 6, blue curve); in contrast, 82Eso1PDL1 co-cultured with Eso1-TCR-T was seen to be significantly inhibited in growth (green curve). The number of target cells co-cultured with TCR-T expressing the enhanced receptor of the present invention (108-TCRT) decreased significantly, indicating that the target cells were killed (FIG. 6, red curve), suggesting that, the killing effect of TCR-T expressing the enhanced receptor was stronger.

Information on key sequences

SEQ ID NO: 1 (PD1-wt)
MQIPQAPWPVVWAVLQLGWRPGWFLDSPDRPWNPPTFSPALLVVTEGDNATFTCS
FSNTSESFVLNWYRMSPSNQTDKLAAFPEDRSQPGQDCRFRVTQLPNGRDFHMSVV
RARRNDSGTYLCGAISLAPKAQIKESLRAELRVTERRAEVPTAHPSPSPRPAGQFQTL
VVGVVGGLLGSLVLLVWVLAVICSRAARGTIGARRTGQPLKEDPSAVPVFSVDYGE
LDFQWREKTPEPPVPCVPEQTEYATIVFPSGMGTSSPARRGSADGPRSAQPLRPEDG
HCSWPL

SEQ ID NO: 2 (Mut1/56#)
MQIPQAPWPVVWAVLQLGWRPGWFLDSPDRPWNPPTFSPALLVVTEGDNATFTCS
FSNTSESFVLNWYRMSPSNQTDKLAAFPEDRSQPGQDCRFRVTQLPNGRDFHMSVV
RARRNDSGTYLCGAISLSTPVWIKESLRAELRVTERRAEVPTAH

SEQ ID NO: 3 (Mut2/56#-2)
MQIPQAPWPVVWAVLQLGWRPGWFLDSPDRPWNPPTFSPALLVVTEGDNATFTCS
FSNTSESFVLNWYRMSPSNQTDKLAAFPFLFNLPGQDCRFRVTQLPNGRDFHMSVV
RARRNDSGTYLCGAISLSTPVWIKESLRAELRVTERRAEVPTAH

SEQ ID NO: 4 (Mut3/56#-3)
MQIPQAPWPVVWAVLQLGWRPGWFLDSPDRPWNPPTFSPALLVVTEGDNATFTCS
FSNTSESFVLNWYRMSPSNQTDKLAAFPWMFCFPGQDCRFRVTQLPNGRDFHMSV
VRARRNDSGTYLCGAISLSTPVWIKESLRAELRVTERRAEVPTAH

SEQ ID NO: 5 (Mut4/56#-15)
MQIPQAPWPVVWAVLQLGWRPGWFLDSPDRPWNPPTFSPALLVVTEGDNATFTCS
FSNTSESFVLNWYRMSPSNQTDKLAAFPEDFRNCTQDCRFRVTQLPNGRDFHMSVV
RARRNDSGTYLCGAISLSTPVWIKESLRAELRVTERRAEVPTAH

SEQ ID NO: 6 (Mut5/56#-16)
MQIPQAPWPVVWAVLQLGWRPGWFLDSPDRPWNPPTFSPALLVVTEGDNATFTCS
FSNTSESFVLNWYRMSPSNQTDKLAAFPEDFFTVSQDCRFRVTQLPNGRDFHMSVV
RARRNDSGTYLCGAISLSTPVWIKESLRAELRVTERRAEVPTAH

SEQ ID NO: 7 (Mut6/56#-108)
MQIPQAPWPVVWAVLQLGWRPGWFLDSPDRPWNPPTFSPALLVVTEGDNATFTCS
FSNTSESFVLNWYRMSPSNQTDKLAAFPEDRINRSQDCRFRVTQLPNGRDFHMSVV
RARRNDSGTYLCGAISLSTPVWIKESLRAELRVTERRAEVPTAH

SEQ ID NO: 8 (Mut7/56#-2-129A132A)
MQIPQAPWPVVWAVLQLGWRPGWFLDSPDRPWNPPTFSPALLVVTEGDNATFTCS
FSNTSESFVLNWYRMSPSNQTDKLAAFPFLFNLPGQDCRFRVTQLPNGRDFHMSVV
RARRNDSGTYLCGAISLATPAWIKESLRAELRVTERRAEVPTAH

SEQ ID NO: 9 (Mut8/56#-15-38)
MQIPQAPWPVVWAVLQLGWRPGWFLDSPDRPWNPPTFSPALLVVTEGDNATFTCS
FSNTSESFVLNWYRMSPSNQTDKLAAFPEDFRNCTQDCRFRVTQLPNGRDFHMSVV
RARRNDSGTYLCGAISLGTPAWIKESLRAELRVTERRAEVPTAH

SEQ ID NO: 10 (Mut9/56#-15-61)

MQIPQAPWPVVWAVLQLGWRPGWFLDSPDRPWNPPTFSPALLVVTEGDNATFTCS
FSNTSESFVLNWYRMSPSNQTDKLAAFPEDFRNCTQDCRFRVTQLPNGRDFHMSVV
RARRNDSGTYLCGAISLATPGWIKESLRAELRVTERRAEVPTAH

SEQ ID NO: 11 (Mut10/56#-15-69)
MQIPQAPWPVVWAVLQLGWRPGWFLDSPDRPWNPPTFSPALLVVTEGDNATFTCS
FSNTSESFVLNWYRMSPSNQTDKLAAFPEDFRNCTQDCRFRVTQLPNGRDFHMSVV
RARRNDSGTYLCGAISLQTPAWIKESLRAELRVTERRAEVPTAH

SEQ ID NO: 12 (Mut11/56#-16-68)
MQIPQAPWPVVWAVLQLGWRPGWFLDSPDRPWNPPTFSPALLVVTEGDNATFTCS
FSNTSESFVLNWYRMSPSNQTDKLAAFPEDFFTVSQDCRFRVTQLPNGRDFHMSVV
RARRNDSGTYLCGAISLYTPAWIKESLRAELRVTERRAEVPTAH

SEQ ID NO: 13 PD1_49#
MQIPQAPWPVVWAVLQLGWRPGWFLDSPDRPWNPPTFSPALLVVTEGDNATFTCS
FSNTSESFVLNWYRMSPSNQTDKLAAFPEDRSQPGQDCRFRVTQLPNGRDFHMSVV
RARRNDSGTYLCGAISLHFRFNIKESLRAELRVTERRAEVPTAH

SEQ ID NO: 14 PD1_110#
MQIPQAPWPVVWAVLQLGWRPGWFLDSPDRPWNPPTFSPALLVVTEGDNATFTCS
FSNTSESFVLNWYRMSPSNQTDKLAAFPEDRSQPGQDCRFRVTQLPNGRDFHMSVV
RARRNDSGTYLCGAISLSEPIVIKESLRAELRVTERRAEVPTAH

SEQ ID NO: 15 PD1_49#_93
MQIPQAPWPVVWAVLQLGWRPGWFLDSPDRPWNPPTFSPALLVVTEGDNATFTCS
FSNTSESFVLNWYRMSPSNQTDKLAAFPEDRLCVFQDCRFRVTQLPNGRDFHMSVV
RARRNDSGTYLCGAISLHFRFNIKESLRAELRVTERRAEVPTAH

SEQ ID NO: 16 PD1_49#_260
MQIPQAPWPVVWAVLQLGWRPGWFLDSPDRPWNPPTFSPALLVVTEGDNATFTCS
FSNTSESFVLNWYRMSPSNQTDKLAAFPEDSFEVRQDCRFRVTQLPNGRDFHMSVV
RARRNDSGTYLCGAISLHFRFNIKESLRAELRVTERRAEVPTAH

SEQ ID NO: 17 PD1_110#_48
MQIPQAPWPVVWAVLQLGWRPGWFLDSPDRPWNPPTFSPALLVVTEGDNATFTCS
FSNTSESFVLNWYRMSPSNQTDKLAAFPEDLYLKYQDCRFRVTQLPNGRDFHMSVV
RARRNDSGTYLCGAISLSEPIVIKESLRAELRVTERRAEVPTAH

SEQ ID NO: 18 56#-15-7
MQIPQAPWPVVWAVLQLGWRPGWFLDSPDRPWNPPTFSPALLVVTEGDNATFTCS
FSNTSESFVLNWYRMSPSNQTDKLAAFPEDFRNCTQDCRFRVTQLPNGRDFHMSVV
RARRNDSGTYLCGAISLWTPMWIKESLRAELRVTERRAEVPTAH

SEQ ID NO: 19 56#-16-12
MQIPQAPWPVVWAVLQLGWRPGWFLDSPDRPWNPPTFSPALLVVTEGDNATFTCS
FSNTSESFVLNWYRMSPSNQTDKLAAFPEDFFTVSQDCRFRVTQLPNGRDFHMSVV
RARRNDSGTYLCGAISLHTPFWIKESLRAELRVTERRAEVPTAH

SEQ ID NO: 20 56#-16-13
MQIPQAPWPVVWAVLQLGWRPGWFLDSPDRPWNPPTFSPALLVVTEGDNATFTCS
FSNTSESFVLNWYRMSPSNQTDKLAAFPEDFFTVSQDCRFRVTQLPNGRDFHMSVV
RARRNDSGTYLCGAISLGTPFWIKESLRAELRVTERRAEVPTAH

SEQ ID NO: 21 56#-16-50
MQIPQAPWPVVWAVLQLGWRPGWFLDSPDRPWNPPTFSPALLVVTEGDNATFTCS
FSNTSESFVLNWYRMSPSNQTDKLAAFPEDFFTVSQDCRFRVTQLPNGRDFHMSVV
RARRNDSGTYLCGAISLITPGWIKESLRAELRVTERRAEVPTAH

SEQ ID NO: 22 56#-108-51
MQIPQAPWPVVWAVLQLGWRPGWFLDSPDRPWNPPTFSPALLVVTEGDNATFTCS
FSNTSESFVLNWYRMSPSNQTDKLAAFPEDRINRSQDCRFRVTQLPNGRDFHMSVV
RARRNDSGTYLCGAISLTTPFWIKESLRAELRVTERRAEVPTAH

SEQ ID NO: 23 56#-108-60
MQIPQAPWPVVWAVLQLGWRPGWFLDSPDRPWNPPTFSPALLVVTEGDNATFTCS
FSNTSESFVLNWYRMSPSNQTDKLAAFPEDRINRSQDCRFRVTQLPNGRDFHMSVV
RARRNDSGTYLCGAISLVTPAWIKESLRAELRVTERRAEVPTAH

SEQ ID NO: 24 56#-108AA
MQIPQAPWPVVWAVLQLGWRPGWFLDSPDRPWNPPTFSPALLVVTEGDNATFTCS
FSNTSESFVLNWYRMSPSNQTDKLAAFPEDRINRSQDCRFRVTQLPNGRDFHMSVV
RARRNDSGTYLCGAISLATPAWIKESLRAELRVTERRAEVPTAH

SEQ ID NO: 25 56#-15AA
MQIPQAPWPVVWAVLQLGWRPGWFLDSPDRPWNPPTFSPALLVVTEGDNATFTCS
FSNTSESFVLNWYRMSPSNQTDKLAAFPEDFRNCTQDCRFRVTQLPNGRDFHMSVV
RARRNDSGTYLCGAISLATPAWIKESLRAELRVTERRAEVPTAH

SEQ ID NO: 26 56#-16AA
MQIPQAPWPVVWAVLQLGWRPGWFLDSPDRPWNPPTFSPALLVVTEGDNATFTCS
FSNTSESFVLNWYRMSPSNQTDKLAAFPEDFFTVSQDCRFRVTQLPNGRDFHMSVV
RARRNDSGTYLCGAISLATPAWIKESLRAELRVTERRAEVPTAH

## Claims

1. A PD-1 variant, **characterized in that**:
(1) the PD-1 variant is capable of binding to a human PD-L1 polypeptide as shown in SEQ ID NO: 28; and
(2) the PD-1 variant does not bind to one or more anti-PD-1 antibodies selected from the group consisting of Sintilimab, Nivolumab, Camrelizumab, Pemrolizumab, and Toripalimab.

2. The PD-1 variant according to claim 1, wherein as compared to the wild-type human PD-1 polypeptide shown in SEQ ID NO: 1, the PD-1 variant has a mutation at one or more of the following amino acid positions: E84, D85, R86, S87, Q88, P89, G90, A129, P130, K131, A132, and Q133, wherein the amino acid positions are numbered according to the amino acid sequence shown in SEQ ID NO: 1.

3. The PD-1 variant according to claims 1 or 2, wherein as compared to the wild-type human PD-1 polypeptide shown in SEQ ID NO: 1, the PD-1 variant has one or more amino acid mutations selected from the group consisting of E84W, E84F; D85L, D85M; R86F; S87N, S87C, S87R, S87F, S87I, S87L, S87Y; Q88L, Q88F, Q88N, Q88T, Q88C, Q88E; P89C, P89V, P89R, P89K; G90T, G90S, G90F, G90R, G90Y; A129S, A129G, A129Q, A129Y, A129H, A129W, A129V, A129T, A129I; P130T, P130F, P130E; K131P, K131R; A132V, A132G, A132F, A132I, A132M; and Q133W, Q133N, Q133V.

4. The PD-1 variant according to claims 2 or 3, wherein as compared to the wild-type human PD-1 polypeptide shown in SEQ ID NO: 1, the PD-1 variant has a mutation at one or more amino acid positions selected from E84, D85, R86, S87, Q88, P89, and G90, and has a mutation at one or more amino acid positions selected from A129, P130, K131, A132, and Q133.

5. The PD-1 variant according to any one of claims 1 to 4, wherein as compared to the wild-type human PD-1 polypeptide shown in SEQ ID NO: 1, the PD-1 variant comprises, or comprises only, a combination of amino acid mutations selected from any one of the following groups (1) to (25):
(1) A129S, P130T, K131P, A132V, and Q133W;
(2) E84F, D85L, R86F, S87N, Q88L, A129S, P130T, K131P, A132V, and Q133W;
(3) E84W, D85M, R86F, S87C, Q88F, A129S, P130T, K131P, A132V, and Q133W;
(4) R86F, S87R, Q88N, P89C, G90T, A129S, P130T, K131P, A132V, and Q133W;
(5) R86F, S87F, Q88T, P89V, G90S, A129S, P130T, K131P, A132V, and Q133W;
(6) S87I, Q88N, P89R, G90S, A129S, P130T, K131P, A132V, and Q133W;
(7) E84F, D85L, R86F, S87N, Q88L, P130T, K131P, and Q133W;
(8) R86F, S87R, Q88N, P89C, G90T, A129G, P130T, K131P, and Q133W;
(9) R86F, S87R, Q88N, P89C, G90T, P130T, K131P, A132G, and Q133W;
(10) R86F, S87R, Q88N, P89C, G90T, A129Q, P130T, K131P, and Q133W; or
(11) R86F, S87F, Q88T, P89V, G90S, A129Y, P130T, K131P, and Q133W;
(12) A129H, P130F, K131R, A132F, and Q133N;
(13) A129S, P130E, K131P, A132I, and Q133V;
(14) S87L, Q88C, P89V, G90F, A129H, P130F, K131R, A132F, and Q133N;
(15) R86S, S87F, Q88E, P89V, G90R, A129H, P130F, K131R, A132F, and Q133N;
(16) R86L, S87Y, Q88L, P89K, G90Y, A129S, P130E, K131P, A132I, and Q133V;
(17) R86F, S87R, Q88N, P89C, G90T, A129W, P130T, K131P, A132M, and Q133W;
(18) R86F, S87F, Q88T, P89V, G90S, A129H, P130T, K131P, A132F, and Q133W;
(19) R86F, S87F, Q88T, P89V, G90S, A129G, P130T, K131P, A132F, and Q133W;
(20) R86F, S87F, Q88T, P89V, G90S, A129I, P130T, K131P, A132G, and Q133W;
(21) S87I, Q88N, P89R, G90S, A129T, P130T, K131P, A132F, and Q133W;
(22) S87I, Q88N, P89R, G90S, A129V, P130T, K131P, and Q133W;
(23) S87I, Q88N, P89R, G90S, P130T, K131P, and Q133W;
(24) R86F, S87R, Q88N, P89C, G90T, P130T, K131P, and Q133W; and
(25) R86F, S87F, Q88T, P89V, G90S, P130T, K131P, and Q133W.

6. The PD-1 variant according to any one of claims 1 to 5, wherein the PD-1 variant does not comprise an intracellular region, or does not comprise both a transmembrane region and an intracellular region.

7. The PD-1 variant according to any one of claims 1 to 6, wherein the variant comprises the amino acid sequence as shown in any one of SEQ ID NOs: 2-26 or an amino acid sequence having at least 85% sequence identity to the variant comprises the amino acid sequence as shown in any one of SEQ ID NOs: 2-26, or is composed of the amino acid sequence as shown in any one of SEQ ID NOs: 2-26 or an amino acid sequence having at least 85% sequence identity to the amino acid sequence as shown in any one of SEQ ID NOs: 2-26.

8. The PD-1 variant according to any one of claims 1 to 7, wherein the PD-1 variant does not bind to at least two, preferably at least three, anti-PD-1 antibodies selected from the group consisting of Sintilimab, Nivolumab, Camrelizumab, Pemrolizumab, and Toripalimab.

9. A fusion protein comprising a PD-1 variant according to any one of claims 1 to 8.

10. The fusion protein according to claim 9, further comprising an immunoglobulin Fc fragment, preferably, the immunoglobulin Fc fragment is derived from a mammal, preferably a human.

11. The fusion protein according to claim 9, wherein the fusion protein comprises the PD-1 variant as an extracellular domain, wherein the PD-1 variant does not comprise an intracellular region, and the fusion protein further comprises an intracellular signal transduction domain derived from a co-stimulatory molecule, and the fusion protein optionally comprises a transmembrane region of PD-1.

12. An isolated nucleic acid molecule encoding the PD-1 variant according to any one of claims 1 to 8, or the fusion protein according to any one of claims 9 to 11.

13. The nucleic acid molecule according to claim 12 comprising a nucleotide sequence as shown in any one of SEQ ID NOs: 56-61, or a nucleotide sequence having at least 85% homology to the nucleotide sequence as shown in any one of SEQ ID NOs: 56-61.

14. An expression vector comprising the isolated nucleic acid molecule according to claims 12 or 13.

15. A host cell comprising the isolated nucleic acid molecule according to claims 12 or 13, or the expression vector according to claim 14.

16. A pharmaceutical composition comprising (1) the PD-1 variant according to any one of claims 1 to 8, or the fusion protein according to any one of claims 9 to 11, and (2) a pharmaceutically acceptable carrier.

17. Use of the PD-1 variant according to any one of claims 1 to 8 or the fusion protein according to any one of claims 9 to 11 in the preparation of a drug for the treatment of cancer.

18. A drug combination comprising (1) the PD-1 variant according to any one of claims 1 to 8, or the fusion protein according to any one of claims 9 to 11, and (2) an anti-PD-1 antibody; wherein the anti-PD-1 variant does not bind to the anti-PD-1 antibody.

19. A cell modified to express the fusion protein according to any one of claims 9 to 11.

20. The cell according to claim 9, wherein the cell is an immune cell, and prior to being modified, the cell is derived from peripheral blood mononuclear cells or is a tumor infiltrating lymphocyte.
